(19) Europäisches Patentamt / European Patent Office / Office européen des brevets

(11) EP 4 653 012 A1

(12) **EUROPEAN PATENT APPLICATION**
published in accordance with Art. 153(4) EPC

(43) Date of publication:
**26.11.2025 Bulletin 2025/48**

(21) Application number: **24744394.8**

(22) Date of filing: **19.01.2024**

(51) International Patent Classification (IPC):
***A61K 39/395*** *(2006.01)* ***A61K 47/68*** *(2017.01)*
***A61K 9/19*** *(2006.01)* ***A61K 47/18*** *(2017.01)*
***A61P 35/00*** *(2006.01)*

(52) Cooperative Patent Classification (CPC):
**A61K 9/19; A61K 39/395; A61K 47/18; A61K 47/68; A61P 35/00**

(86) International application number:
**PCT/CN2024/073229**

(87) International publication number:
**WO 2024/153224 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.01.2023 CN 202310076956**

(71) Applicants:
- **Jiangsu Hengrui Pharmaceuticals Co., Ltd.**
**Lianyungang, Jiangsu 222047 (CN)**
- **Shanghai Hengrui Pharmaceutical Co., Ltd.**
**Shanghai 200245 (CN)**

(72) Inventors:
- **WU, Tingting**
**Shanghai 200245 (CN)**
- **DU, Min**
**Shanghai 200245 (CN)**

(74) Representative: **Regimbeau**
**20, rue de Chazelles**
**75847 Paris Cedex 17 (FR)**

(54) **PHARMACEUTICAL COMPOSITION CONTAINING ERIBULIN DERIVATIVE DRUG CONJUGATE**

(57) The present disclosure relates to a pharmaceutical composition containing an Eribulin derivative drug conjugate. Specifically, the present disclosure relates to a pharmaceutical composition, comprising an antibody-drug conjugate and a buffering agent. The antibody-drug conjugate has a structure as shown in Formula I, wherein Ab is Pertuzumab. The pharmaceutical composition of the present disclosure has good stability.

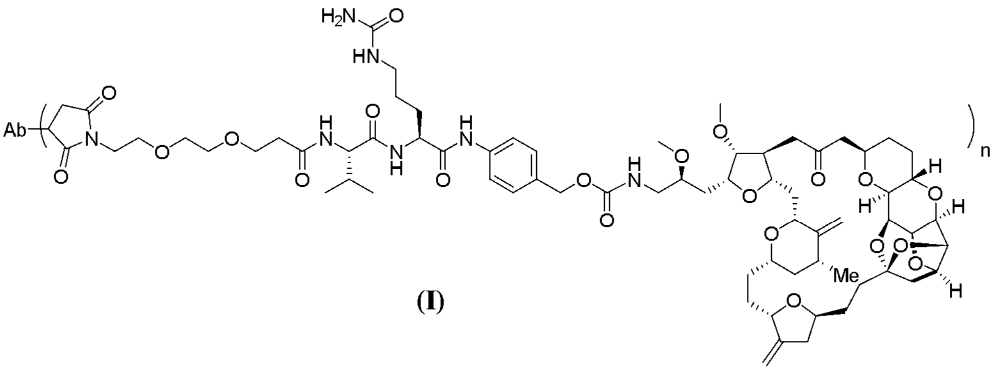

(I)

EP 4 653 012 A1

## Description

### TECHNICAL FIELD

The present disclosure relates to the field of pharmaceutical formulations, and particularly to a pharmaceutical composition comprising an eribulin derivative drug conjugate.

### BACKGROUND

An antibody-drug conjugate (ADC) links a monoclonal antibody or an antibody fragment to a biologically active drug via a stable chemical linker compound, fully exploiting the binding specificity of the antibody to surface antigens of normal cells and tumor cells and the high efficiency of the drug, and also avoiding the former's disadvantage of having a poor therapeutic effect, the latter's disadvantage of having serious toxic side effects, and the like. This means that antibody-drug conjugates can bind to tumor cells precisely and reduce the impact on normal cells compared to conventional chemotherapeutic drugs.

Microtubules are potent filamentous cytoskeletal proteins associated with a variety of cellular functions including intracellular migration and transport, cell signaling, and maintenance of cell shape. Microtubules also play a critical role in mitotic cell division by forming the mitotic spindle required for chromosomes to divide into two daughter cells. The biological functions of microtubules in all cells are regulated in large part by their polymerization dynamics, and the polymerization of microtubules occurs by the reversible, non-covalent addition of $\alpha$ and $\beta$ tubulin dimers at both ends of microtubules. This dynamic behavior and the resulting control of microtubule length are essential for proper function of the mitotic spindle. Even minor changes in microtubule dynamics involve the spindle checkpoint, inhibit cell cycle progression at mitosis, and subsequently cause cell death. Since cancer cells divide rapidly, they are generally more sensitive to compounds that bind to tubulin and disrupt their normal functions than normal cells. Therefore, tubulin inhibitors and antibody-drug conjugates thereof are expected to be a promising class of drugs for the treatment of cancer.

ADCs have a more complex heterogeneous structure than antibodies, which presents greater challenges in developing ADC formulations for therapeutic purposes.

### SUMMARY

The present disclosure provides a pharmaceutical composition comprising an antibody-drug conjugate and a buffer, wherein the antibody-drug conjugate has a structure represented by formula I:

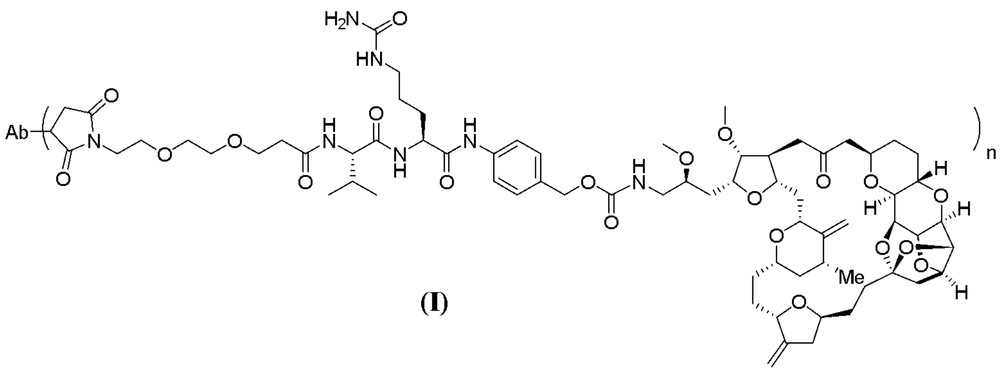

(I)

,

wherein:

Ab is pertuzumab;
n is 1 to 10;
the buffer is selected from the group consisting of a citrate buffer, a histidine buffer, and a succinate buffer.

In some embodiments, the buffer is selected from the group consisting of histidine-histidine hydrochloride, citric acid-sodium citrate, and succinic acid-sodium succinate.

In some embodiments, the range of drug loading (n) may be the average number of cytotoxic drugs bound per antibody pertuzumab; non-limiting examples include the average number of cytotoxic drugs bound per antibody being 1, 2, 3, 4, 5, 6, 7, 8, 9, 10, 11, and 12, as well as any range between these point values. For example, the range of drug loading may be 2-8, 2-7, 2-6, 2-5, 2-4, 3-4, 3-5, 3.5-4.7, 5-6, 5-7, 5-8, and 6-8. Illustratively, the drug loading (n) may be an average

of 1, 2, 3, 4, 5, 6, 7, 8, 9, or 10. n is a decimal or integer. In some embodiments, n is 1 to 8, or 3 to 5.

In some embodiments, the pharmaceutical composition has a pH of 3.5 to 5.5; non-limiting examples include 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, and 5.5, as well as any range between these point values. In some embodiments, the pH is 3.5 to 5.0, or the pH is 3.7 to 4.7.

In some embodiments, the buffer in the pharmaceutical composition is at a concentration of 5 mM to 50 mM; non-limiting examples include 5 mM, 10 mM, 12 mM, 15 mM, 16 mM, 17 mM, 18 mM, 19 mM, 20 mM, 30 mM, 40 mM, and 50 mM, as well as any range between these point values; in some embodiments, the buffer is at a concentration of 10 mM to 50 mM; in some embodiments, the buffer is at a concentration of 20 mM to 40 mM; in some embodiments, the buffer is at a concentration of 30 mM.

In some embodiments, the pharmaceutical composition further comprises a surfactant. The surfactant may be selected from the group consisting of polysorbate, poloxamer, poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramidopropyl-betaine, cocamidopropyl-betaine, linoleamidopropyl-betaine, myristamidopropyl-betaine, palmitamidopropyl-betaine, isostearamidopropyl-betaine, myristamidopropyl-dimethylamine, palmitamidopropyl-dimethylamine, isostearamidopropyl-dimethylamine, sodium methyl cocoyl, sodium methyl oleyl taurate, polyethylene glycol, polypropylene glycol, copolymers of ethylene and propylene glycol, etc.In some embodiments, the surfactant is poloxamer or polysorbate, such as poloxamer 188, polysorbate 20, or polysorbate 80.

In some embodiments, the surfactant in the pharmaceutical composition is at a concentration of 0.01 mg/mL to 1.0 mg/mL, or 0.1 mg/mL to 0.8 mg/mL, or 0.3 mg/mL to 0.8 mg/mL; in some embodiments, the surfactant is at a concentration of 0.6 mg/mL; non-limiting examples include 0.02 mg/mL, 0.05 mg/mL, 0.1 mg/mL, 0.15 mg/mL, 0.2 mg/mL, 0.25 mg/mL, 0.3 mg/mL, 0.35 mg/mL, 0.4 mg/mL, 0.45 mg/mL, 0.5 mg/mL, 0.6 mg/mL, 0.7 mg/mL, and 0.8 mg/mL, as well as any range between these point values.

In some embodiments, the aforementioned pharmaceutical composition further comprises a sugar. "Sugar" in the present disclosure includes the general composition $(CH_2O)_n$ and derivatives thereof, including monosaccharides, disaccharides, trisaccharides, polysaccharides, sugar alcohols, reducing sugars, non-reducing sugars, etc. The sugar may be selected from the group consisting of glucose, sucrose, trehalose, α,α-trehalose dihydrate, lactose, fructose, maltose, dextran, glycerin, erythritol, glycerol, arabitol, sylitol, sorbitol, mannitol, melibiose, melezitose, raffinose, mannotriose, stachyose, maltose, lactulose, maltulose, glucitol, maltitol, lactitol, iso-maltulose, etc. In some embodiments, the sugar is sucrose.

In some embodiments, the sugar in the aforementioned pharmaceutical composition is at a concentration of 25 mg/mL to 80 mg/mL, or 30 mg/mL to 50 mg/mL; non-limiting examples include 25 mg/mL, 30 mg/mL, 35 mg/mL, 40 mg/mL, 45 mg/mL, 50 mg/mL, 55 mg/mL, 60 mg/mL, 70 mg/mL, and 80 mg/mL, as well as any range between these point values. In some embodiments, the concentration is 40 mg/mL.

In some embodiments, the aforementioned pharmaceutical composition further comprises an amino acid, such as glycine.

In some embodiments, the amino acid in the aforementioned pharmaceutical composition is at a concentration of 6 mg/mL to 15 mg/mL, e.g., 7 mg/mL to 11 mg/mL, or 7 mg/mL to 10 mg/mL; non-limiting examples include 6 mg/mL, 6.5 mg/mL, 7 mg/mL, 7.2 mg/mL, 7.6 mg/mL, 7.8 mg/mL, 8 mg/mL, 8.5 mg/mL, 9 mg/mL, 10 mg/mL, 10.2 mg/mL, 11 mg/mL, 12 mg/mL, 13 mg/mL, 14 mg/mL, and 15 mg/mL, as well as any range between these point values. In some embodiments, the concentration is 9 mg/mL. In some embodiments, the antibody-drug conjugate in the pharmaceutical composition is at a concentration of 1 mg/mL to 100 mg/mL on a protein concentration basis; non-limiting examples include 1 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, 25 mg/mL, 26 mg/mL, 27 mg/mL, 28 mg/mL, 29 mg/mL, 30 mg/mL, 40 mg/mL, 50 mg/mL, 60 mg/mL, 70 mg/mL, 80 mg/mL, 90 mg/mL, and 100 mg/mL, as well as any range between these point values; in some embodiments, the antibody-drug conjugate is at a concentration of 10 mg/mL to 30 mg/mL, e.g., 20 mg/mL, on a protein concentration basis. Specifically, non-limiting examples include 20.1 mg/mL, 20.2 mg/mL, 20.3 mg/mL, 20.4 mg/mL, 20.5 mg/mL, 20.6 mg/mL, 20.7 mg/mL, 20.8 mg/mL, 20.81 mg/mL, 20.82 mg/mL, 20.83 mg/mL, 20.84 mg/mL, 20.85 mg/mL, 20.86 mg/mL, 20.87 mg/mL, 20.88 mg/mL, 20.89 mg/mL, 20.9 mg/mL, 20.91 mg/mL, 20.92 mg/mL, 20.93 mg/mL, 20.94 mg/mL, 20.95 mg/mL, 20.96 mg/mL, 20.97 mg/mL, 20.98 mg/mL, 20.99 mg/mL, and 21 mg/mL, as well as any range between these point values. The "on a protein concentration basis" refers to on the basis of the concentration of the antibody moiety in the antibody-drug conjugate. In some embodiments, the pharmaceutical composition comprises:

(a) on a protein concentration basis, 1 mg/mL to 100 mg/mL said antibody-drug conjugate, (b) 0.1 mg/mL to 0.8 mg/mL poloxamer or polysorbate, (c) 30 mg/mL to 50 mg/mL sucrose, (d) 6 mg/mL to 15 mg/mL glycine, and (e) 10 mM to 50 mM succinic acid-sodium succinate buffer; the pharmaceutical composition having a pH of 3.5 to 5.5. In some embodiments, the pharmaceutical composition comprises:

(a) on a protein concentration basis, 1 mg/mL to 50 mg/mL said antibody-drug conjugate, (b) 0.2 mg/mL to 0.8 mg/mL

poloxamer or polysorbate, (c) 30 mg/mL to 50 mg/mL sucrose, (d) 6 mg/mL to 15 mg/mL glycine, and (e) 20 mM to 40 mM succinic acid-sodium succinate buffer; the pharmaceutical composition having a pH of 3.7 to 4.7. In some embodiments, the pharmaceutical composition comprises:

(a) on a protein concentration basis, 20 mg/mL said antibody-drug conjugate, (b) 0.6 mg/mL poloxamer 188 or 0.2 mg/mL polysorbate 80, (c) 40 mg/mL sucrose, (d) 9 mg/mL glycine, and (e) 30 mM succinic acid-sodium succinate buffer; the pharmaceutical composition having a pH of 4.2.

In some embodiments, the pharmaceutical composition according to any one of the foregoing is a liquid formulation. The liquid formulation or the reconstituted formulation of the present disclosure has relatively good stability. Further, stable liquid formulations include liquid formulations that exhibit desirable features after 1 month of storage at temperatures including 40 °C.

The present disclosure further provides a freeze-dried formulation comprising an antibody-drug conjugate, wherein the formulation can form the pharmaceutical composition described above after being reconstituted.

The present disclosure further provides a method for preparing a freeze-dried formulation comprising an antibody-drug conjugate, the method comprising a step of lyophilizing the pharmaceutical composition described above.

In some embodiments, the freeze-dried formulation is stable at 40 °C for at least 7 days, at least 14 days, at least 28 days, or at least 30 days.

The present disclosure further provides a freeze-dried formulation comprising an antibody-drug conjugate, wherein the freeze-dried formulation is obtained by lyophilizing the pharmaceutical composition of the antibody-drug conjugate described above.

The present disclosure further provides a reconstituted solution comprising an antibody-drug conjugate, wherein the reconstituted solution is prepared by reconstituting the freeze-dried formulation described above.

The present disclosure further provides a method for preparing the reconstituted solution described above, the method comprising a step of reconstituting the aforementioned freeze-dried formulation with a solution selected from the group consisting of, but not limited to, water for injection, normal saline, and glucose solution.

The present disclosure further provides a product comprising a container containing the pharmaceutical composition, the freeze-dried formulation, or the reconstituted solution described above. In some embodiments, the container is a tubular injection vial made of neutral borosilicate glass.

The present disclosure further provides use of the aforementioned pharmaceutical composition or freeze-dried formulation or reconstituted solution or product in the manufacture of a medicament for treating or preventing a tumor.

The present disclosure further provides a method for treating a disease, the method comprising administering to a patient in need thereof the aforementioned pharmaceutical composition or freeze-dried formulation or reconstituted solution or product.

The present disclosure further provides the aforementioned pharmaceutical composition, or freeze-dried formulation, or reconstituted solution, or product as a medicament for use in treating a disease.

The present disclosure further provides the aforementioned pharmaceutical composition, or freeze-dried formulation, or reconstituted solution, or product as a medicament for use in treating a tumor.

The present disclosure further provides use of the aforementioned pharmaceutical composition, or freeze-dried formulation, or reconstituted solution, or product as a medicament in the manufacture of a medicament for treating a tumor.

In some embodiments, the tumor is a cancer associated with expression of a domain II of HER2.

In some embodiments, the tumor is selected from the group consisting of breast cancer, ovarian cancer, cervical cancer, uterine cancer, prostate cancer, renal cancer, urethral cancer, bladder cancer, liver cancer, gastric cancer, endometrial cancer, salivary gland cancer, esophageal cancer, melanoma, neuroglioma, neuroblastoma, sarcoma, lung cancer, colon cancer, rectal cancer, colorectal cancer, leukemia, bone cancer, skin cancer, thyroid cancer, pancreatic cancer, and lymphoma.

The heavy and light chain variable region sequences of pertuzumab are as follows:

Light chain variable region

DIQMTQSPSSLSASVGDRVTITCKASQDVSIGVAWYQQKPGKAPKLLIYSASYRY
TGVPSRFSGSGSGTDFTLTISSLQPEDFATYYCQQYYIYPYTFGQGTKVEIK

SEQ ID NO: 1

Heavy chain variable region

EVQLVESGGGLVQPGGSLRLSCAASGFTFTDYTMDWVRQAPGKGLEWVADVN
PNSGGSIYNQRFKGRFTLSVDRSKNTLYLQMNSLRAEDTAVYYCARNLGPSFYF
DYWGQGTLVTVSS

SEQ ID NO: 2

The sequence of pertuzumab is as follows:

Light chain

DIQMTQSPSSLSASVGDRVTITCKASQDVSIGVAWYQQKPGKAPKLLIYSASYRY
TGVPSRFSGSGSGTDFTLTISSLQPEDFATYYCQQYYIYPYTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO: 3

Heavy chain

EVQLVESGGGLVQPGGSLRLSCAASGFTFTDYTMDWVRQAPGKGLEWVADVN
PNSGGSIYNQRFKGRFTLSVDRSKNTLYLQMNSLRAEDTAVYYCARNLGPSFYF
DYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVM

HEALHNHYTQKSLSLSPGK

SEQ ID NO: 4

As is well known to those skilled in the art, one, some, or all of the features of the various embodiments described in the present disclosure may be further combined to form other embodiments of the present disclosure. The above embodiments of the present disclosure and other embodiments obtained by combination are further illustrated through the following detailed description.

**Detailed Description of the Disclosure**

The present disclosure provides a pharmaceutical composition that favors production and administration and has stable properties. Undesirable instability may include any one or more of aggregation, deamidation (e.g., Asn deamidation), oxidation (e.g., Met oxidation), isomerization (e.g., Asp isomerization), clipping/hydrolysis/fragmentation (e.g., hinge region fragmentation), succinimide formation, unpaired cysteines, dissociation of toxins, and the like. Specifically, the pharmaceutical composition described in the present disclosure comprises an antibody-drug conjugate and a buffer.

**Terminology**

In order to facilitate the understanding of the present disclosure, certain technical and scientific terms are

specifically defined below. Unless otherwise specifically defined herein, all other technical and scientific terms used herein have the meanings generally understood by those of ordinary skill in the art to which the present disclosure pertains.

"Antibody-drug conjugate (ADC)" means that an antibody is linked to a biologically active cytotoxin or a small-molecule drug with cell killing activity by a linker unit.

"Drug loading" is also referred to as the drug-to-antibody ratio (DAR), i.e., the average number of drugs conjugated per antibody in the ADC. It may range, for example, from 1 to 10 drugs conjugated per antibody, and in certain embodiments, from 1 to 8 drugs conjugated per antibody; preferably, it is selected from the group consisting of 2-8, 2-7, 2-6, 2-5, 2-4, 3-4, 3-5, 5-6, 5-7, 5-8, and 6-8. Illustratively, the drug loading may be an average of 1, 2, 3, 4, 5, 6, 7, 8, 9, or 10. The ADC general formula of the present disclosure includes a set of antibodies conjugated to drugs within a certain range as described above. In an embodiment of the present disclosure, the drug loading may be represented by n. Drug loading can be determined by conventional methods such as UV/visible spectroscopy, mass spectrometry, ELISA assay and HPLC.

The term "linker unit" or "linker fragment" refers to a chemical structure fragment or bond that is linked to an antibody or an antigen-binding fragment thereof at one end and to a drug at the other end, and it may also be linked to a drug after being linked to another linker.

The linker comprises a stretcher, a spacer, and an amino acid unit and may be synthesized using methods known in the art, such as those described in US20050238649A1. The linker may be a "cleavable linker" favorable for the release of drugs in cells. For example, acid-labile linkers (e.g., hydrazones), protease-sensitive (e.g., pepti-dase-sensitive) linkers, photolabile linkers, dimethyl linkers or disulfide-containing linkers can be used (Chari et al., Cancer Research, 52: 127-131(1992); U.S. Patent No. 5,208,020).

The term "drug linker arm fragment" or "drug-linker fragment" refers to a fragment formed by linking a drug and a linker unit, which can be linked to an antibody through the other end of the linker unit.

The loading of the cytotoxic drug can be controlled using the following non-limiting methods, including:

(1) controlling the molar ratio of the drug linker arm fragment to the monoclonal antibody,
(2) controlling reaction time and temperature, and
(3) selecting different reaction reagents.

The three-letter and single-letter codes for amino acids used in the present disclosure are as described in J. Biol. Chem, 243, p3558 (1968).

"Antibody" described in the present disclosure is used in the broadest sense and encompasses a variety of antibody structures, including but not limited to full-length antibodies and antibody fragments (or antigen-binding fragments, or antigen-binding moieties), as long as they exhibit the desired antigen-binding activity. In general, a natural intact antibody is a four-peptide-chain structure formed by linking two identical heavy chains and two identical light chains by interchain disulfide bonds.

The engineered antibodies or antigen-binding fragments of the present disclosure can be prepared and purified by conventional methods. For example, cDNA sequences encoding the heavy and light chains can be cloned and recombined into a GS expression vector. CHO cells can be stably transfected with recombinant immunoglobulin expression vectors. As a more recommended prior art, mammalian expression systems will result in glycosylation of antibodies, particularly at the highly conserved N-terminal site of the Fc region. Positive clones are expanded in a serum-free medium in a bioreactor to produce antibodies. The culture solution with the secreted antibody can be purified by conventional techniques. For example, purification is performed using an A or G Sepharose FF column containing an adjusted buffer. Non-specifically bound components are washed away. The bound antibody is eluted by the pH gradient method, and the antibody fragments are detected by SDS-PAGE and collected. The antibodies can be filtered and concentrated by conventional methods. Soluble mixtures and polymers can also be removed by conventional methods, such as molecular sieves and ion exchange. The resulting products need to be immediately frozen, such as at -70 °C, or lyophilized. "Buffer" refers to a buffer that resists changes in pH by the action of its acid-base conjugate components. Examples of buffers that maintain the pH within an appropriate range include acetate, succinate, gluconate, histidine, oxalate, lactate, phosphate, citrate, tartrate, fumarate, glycylglycine, and other organic acid buffers.

"Histidine buffer" is a buffer comprising histidine. Examples of histidine buffers include histidine-histidine hydrochloride, histidine-histidine acetate, histidine-histidine phosphate, histidine-histidine sulfate, and the like. The histidine-histidine hydrochloride buffer is preferred. The histidine-histidine hydrochloride buffer may be prepared from histidine and hydrochloric acid, or from histidine and histidine hydrochloride.

"Citrate buffer" is a buffer comprising citrate ions. Examples of citrate buffers include citric acid-sodium citrate, citric acid-potassium citrate, citric acid-calcium citrate, citric acid-magnesium citrate, and the like. The preferred citrate buffer is citric acid-sodium citrate.

"Succinate buffer" is a buffer comprising succinate ions. Examples of succinate buffers include succinic acid-sodium succinate, succinic acid-potassium succinate, succinic acid-calcium succinate, and the like. The preferred

succinate buffer is succinic acid-sodium succinate. Illustratively, the succinic acid-sodium succinate may be prepared from succinic acid and sodium hydroxide, or from succinic acid and sodium succinate.

"Phosphate buffer" is a buffer comprising phosphate ions. Examples of phosphate buffers include disodium hydrogen phosphate-sodium dihydrogen phosphate, disodium hydrogen phosphate-potassium dihydrogen phosphate, disodium hydrogen phosphate-citric acid, and the like. The preferred phosphate buffer is disodium hydrogen phosphate-sodium dihydrogen phosphate.

"Acetate buffer" is a buffer comprising acetate ions. Examples of acetate buffers include acetic acid-sodium acetate, histidine-histidine acetate, acetic acid-potassium acetate, acetic acid-calcium acetate, acetic acid-magnesium acetate, and the like. The preferred acetate buffer is acetic acid-sodium acetate.

"Pharmaceutical composition" refers to a mixture containing one or more of the antibody-drug conjugates described herein or physiologically/pharmaceutically acceptable salts or prodrugs thereof, and other chemical components; the other components are, for example, physiologically/pharmaceutically acceptable carriers and excipients. The purpose of the pharmaceutical composition is to maintain the stability of the active ingredient of the antibody and promote the administration to an organism, which facilitates the absorption of the active ingredient, thereby exerting biological activity.

In the present disclosure, "pharmaceutical composition" and "formulation" are not mutually exclusive.

Unless otherwise specified, the solvent in the pharmaceutical composition described in the present disclosure in solution form is water.

"Freeze-dried formulation" refers to a formulation or a pharmaceutical composition obtained by lyophilizing a pharmaceutical composition or a formulation in liquid or solution form *in vacuo*.

The terms "about" and "approximately" as used herein mean that a numerical value is within an acceptable error range for the particular value determined by one of ordinary skill in the art, and the numerical value depends in part on how the value is measured or determined (i.e., the limits of the measurement system). For example, "about" may mean a standard deviation within 1 or more than 1 in each practice in the art. Alternatively, "about" or "substantially comprising" may mean a range of up to 20%. Furthermore, particularly for biological systems or processes, the term may mean up to an order of magnitude or up to 5-fold of a numerical value. Unless otherwise stated, when a specific value is provided in the present application and claims, the meaning of "about" or "substantially comprise" should be assumed to be within an acceptable error range for that specific value.

The values in the present disclosure are instrument measurements or calculated values after instrument measurement, and have a certain degree of error. Generally, ±10% falls within a reasonable error range. It is certainly necessary to consider the context in which the value is used, for example, for the content of the total impurity, the error range of the value after the measurement shall not exceed ±10%, and may be ±9%, ±8%, ±7%, ±6%, ±5%, ±4%, ±3%, ±2% or ±1%, preferably ±5%.

The pharmaceutical composition described in the present disclosure can achieve a stable effect: a pharmaceutical composition in which the antibody-drug conjugate substantially retains its physical and/or chemical stability and/or biological activity after storage; preferably, the pharmaceutical composition substantially retains its physical and chemical stability as well as its biological activity after storage. The storage period is generally selected based on a predetermined shelf life of the pharmaceutical composition. There are a variety of analytical techniques currently available for measuring the stability of a protein or an antibody-drug conjugate, and the stability after storage for a selected period of time at a selected temperature can be measured.

A stable formulation is one in which no significant change is observed under the following conditions: stored at refrigeration temperature (2-8 °C) for at least 3 months, preferably 6 months, and more preferably 1 year. In addition, stable liquid formulations include liquid formulations that exhibit desirable features after storage at temperatures including 25 °C for periods of time including 1 month, 2 months, or 3 months. Further, stable liquid formulations include liquid formulations that exhibit desirable features after storage at temperatures including 40 °C for periods of time including 10 days, 20 days, and 1 month. Typical examples for stability are as follows: generally, no more than about 10%, preferably no more than about 5%, of antibody monomers aggregate or are degraded as measured by SEC-HPLC. The formulation is a pale yellow, nearly colorless and clear liquid, or colorless, or clear to slightly opalescent, by visual analysis. The concentration, pH, and osmolality of the formulation have a change of no more than ±10%. Generally, a decrease of no more than about 10%, preferably no more than about 5% is observed. Generally, aggregation of no more than about 10%, preferably no more than about 5% is formed.

An antibody-drug conjugate "retains its physical stability" in a pharmaceutical formulation if it shows no significant increase in aggregation, precipitation, and/or denaturation upon visual examination of color and/or clarity, or as measured by UV light scattering, size exclusion chromatography (SEC), and dynamic light scattering (DLS). Changes in protein conformation can be evaluated by fluorescence spectroscopy (which determines the protein tertiary structure) and by FTIR spectroscopy (which determines the protein secondary structure).

An antibody-drug conjugate "retains its chemical stability" in a pharmaceutical formulation if it shows no significant chemical change. Chemical stability can be evaluated by detecting and quantifying chemically changed

proteins. Degradation processes that often change the chemical structure of proteins include hydrolysis or clipping (assessed by methods such as size exclusion chromatography and CE-SDS), oxidation (assessed by methods such as peptide mapping in combination with mass spectrometry or MALDI/TOF/MS), deamidation (assessed by methods such as ion-exchange chromatography, capillary isoelectric focusing, peptide mapping, and isoaspartic acid determination), and isomerization (assessed by isoaspartic acid content determination, peptide mapping, etc.).

An antibody-drug conjugate "retains its biological activity" in a pharmaceutical formulation if the biological activity of the antibody-drug conjugate at a given time is within a predetermined range of the biological activity exhibited at the time the pharmaceutical formulation was prepared.

"Optional" or "optionally" means that the event or circumstance subsequently described may, but does not necessarily, occur, and this description includes instances where the event or circumstance occurs or does not occur. For example, "optionally comprising 1-3 antibody heavy chain variable regions" means that the antibody heavy chain variable region of a particular sequence may, but does not necessarily, exist.

"Substituted" means that one or more, preferably up to 5, and more preferably 1 to 3, hydrogen atoms in the group are independently substituted with a corresponding number of substituents. It goes without saying that a substituent is only in its possible chemical position, and those skilled in the art will be able to determine (experimentally or theoretically) possible or impossible substitution without undue effort. For example, it may be unstable when an amino or hydroxy having free hydrogen is bound to a carbon atom having an unsaturated (e.g., olefinic) bond.

For the preparation of conventional pharmaceutical compositions, refer to Chinese Pharmacopoeia.

The term "carrier" used for the drug of the present disclosure refers to a system that can alter the manner in which the drug gets into a human body and the distribution of the drug in the human body, control the release rate of the drug, and deliver the drug to a targeted organ. The drug carrier release and targeted system can reduce drug degradation and loss, reduce side effects, and improve bioavailability. For example, polymeric surfactants that can be used as carriers can self-assemble due to their unique amphiphilic structures to form various forms of aggregates; preferred examples are, e.g., micelles, microemulsions, gels, liquid crystals, and vesicles. The aggregates have the capability of encapsulating drug molecules and have good permeability for membranes, and therefore can be used as excellent drug carriers.

"Administer" and "treat", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids. "Administer" and "treat" can refer to, for example, therapeutic, pharmacokinetic, diagnostic, research, and experimental methods. The treatment of cells comprises contacting a reagent with cells and contacting the reagent with a fluid, where the fluid is in contact with the cells. "Administer" and "treat" also refer to treating, e.g., cells, by reagents, diagnosis, binding compositions or by another cell *in vitro* and *ex vivo.* "Treating", when applied to humans, veterinary or research subjects, refers to therapeutic treatment, preventive or prophylactic measures, and research and diagnostic applications.

"Treatment" refers to administering a therapeutic agent, such as a composition comprising any one of the conjugation compounds of the present disclosure, either internally or externally to a patient with one or more symptoms of a disease on which the therapeutic agent is known to have a therapeutic effect. Typically, the therapeutic agent is administered in an amount effective to alleviate one or more disease symptoms in the patient or population being treated to induce regression of such symptoms or to inhibit the development of such symptoms to any clinically measurable degree. The amount of the therapeutic agent effective to alleviate any specific disease symptom (also known as a "therapeutically effective amount") may vary depending on a variety of factors, such as the disease state, age and body weight of the patient, and the ability of the drug to produce a desired therapeutic effect in the patient. Whether a disease symptom has been alleviated can be evaluated by any clinical testing methods commonly used by doctors or other health care professionals to evaluate the severity or progression of the symptom. Although the embodiments of the present disclosure (e.g., treatment methods or products) may not be effective in alleviating all the target disease symptoms in every patient, as determined according to any statistical testing methods known in the art, such as Student t-test, chi-square test, Mann and Whitney's U test, Kruskal-Wallis test (H test), Jonckheere-Terpstra test, and Wilcoxon test, they should reduce the target disease symptoms in a statistically significant number of patients.

"Effective amount" comprises an amount sufficient to ameliorate or prevent a symptom or disorder of a medical disease. An effective amount also means an amount sufficient to allow or facilitate diagnosis. The effective amount for a particular patient or veterinary subject may vary depending on factors such as the disorder to be treated, the general health of the patient, the method and route and dose of administration, and the severity of side effects. An effective amount may be the maximum dose or administration regimen to avoid significant side effects or toxic effects.

"Exchange" refers to the exchange of a solvent system that solubilizes an antibody protein or antibody-drug conjugate. For example, a high-salt or hypertonic solvent system comprising the antibody protein or antibody-drug conjugate is exchanged, by physical operations, with a buffer system of a stable formulation, such that the antibody protein or antibody-drug conjugate is present in the stable formulation. The physical operations include, but are not limited to, ultrafiltration, dialysis, or reconstitution following centrifugation.

Herein, "pertuzumab" refers to an antibody comprising a light chain variable region and a heavy chain variable

region having amino acid sequences set forth in SEQ ID NOs: 1 and 2, respectively. If pertuzumab is an intact antibody, it preferably comprises a light chain and a heavy chain having amino acid sequences set forth in SEQ ID NOs: 3 and 4, respectively.

## BRIEF DESCRIPTION OF THE DRAWINGS

FIG. 1 is a graph showing the change in tumor volume of N87/16-8 subcutaneous xenograft tumor model mice.
FIG. 2 is a graph showing the change in body weight of N87/16-8 subcutaneous xenograft tumor model mice.
FIG. 3 is a graph showing the change in tumor volume of JIMIT-1 subcutaneous xenograft tumor model mice.
FIG. 4 is a graph showing the change in body weight of JIMIT-1 subcutaneous xenograft tumor model mice.

## DETAILED DESCRIPTION

The present disclosure is further described below with reference to examples, which, however, are not intended to limit the scope of the present disclosure. The experimental methods in the examples of the present disclosure in which specific conditions are not specified are generally performed under conventional conditions, for example, by referring to Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated were commercially available conventional reagents.

The structures of the compounds were determined by nuclear magnetic resonance (NMR) spectroscopy or/and mass spectrometry (MS). The NMR shifts (δ) are given in $10^{-6}$ (ppm). The NMR analyses were performed using a Bruker AVANCE-400 nuclear magnetic resonance instrument, with dimethyl sulfoxide-D6 (DMSO-$d_6$), chloroform-D ($CDCl_3$), and methanol-D4 ($CD_3OD$) as solvents and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS), waters ACQuity UPLC-QD/SQD (manufacturer: waters; MS model: waters ACQuity Qda Detector/waters SQ Detector), and THERMO Ultimate 3000-Q Exactive (manufacturer: THERMO; MS model: THERMO Q Exactive).

The high performance liquid chromatography (HPLC) analyses were performed using Agilent HPLC 1200DAD, Agilent HPLC 1200VWD, and Waters HPLC e2695-2489 high-pressure liquid chromatographs.

The chiral HPLC analyses were performed using an Agilent 1260 DAD high performance liquid chromatograph.

The preparative high performance liquid chromatography used Waters 2545-2767, Waters 2767-SQ Detecor2, Shimadzu LC-20AP, and Gilson GX-281 preparative chromatographs.

The preparative chiral chromatography used a Shimadzu LC-20AP preparative chromatograph.

The CombiFlash preparative flash chromatograph used was Combiflash Rf200 (TELEDYNE ISCO).

Yantai Huanghai HSGF254 or Qingdao GF254 silica gel plates, 0.15-0.2 mm layer thickness, were adopted for thin-layer chromatography (TLC) analysis and 0.4-0.5 mm layer thickness for TLC separation and purification.

Yantai Huanghai silica gel of 200-300 mesh was generally used as a carrier in silica gel column chromatography.

Known starting materials in the present disclosure may be synthesized by using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can all be performed under an argon atmosphere or a nitrogen atmosphere unless otherwise specified.

The argon atmosphere or nitrogen atmosphere means that the reaction flask was connected to a balloon containing about 1 L of argon or nitrogen gas.

The hydrogen atmosphere means that the reaction flask was connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 cycles of vacuumization and hydrogen filling.

The microwave reactions were performed using a CEM Discover-S 908860 microwave reactor.

In the examples, the solutions were aqueous solutions unless otherwise specified.

In the examples, the reaction temperature was room temperature, i.e., 20 °C to 30 °C, unless otherwise specified.

The eluent systems used in the column chromatography purification of the compounds and the developing solvent systems used in the thin-layer chromatography analyses include: A: a system of dichloromethane and isopropanol, B: a system of dichloromethane and methanol, and C: a system of petroleum ether and ethyl acetate. The volume ratio of the solvents was adjusted depending on the polarity of the compound, or by adding a small amount of triethylamine

and an acidic or basic reagent.

The antibody-drug conjugate represented by formula I described herein can be prepared according to the method described in PCT/CN2022/107479.

## I. Preparation of Antibody Conjugate

### Example 1

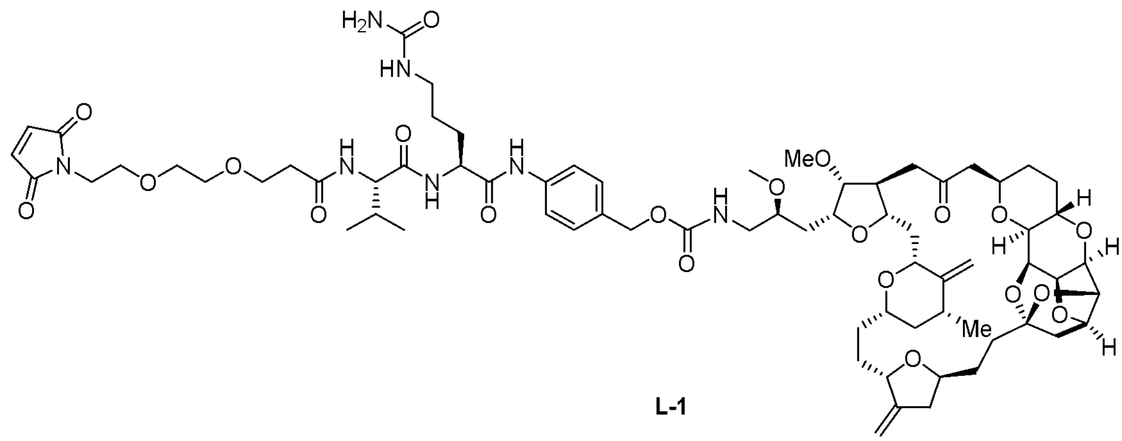

L-1

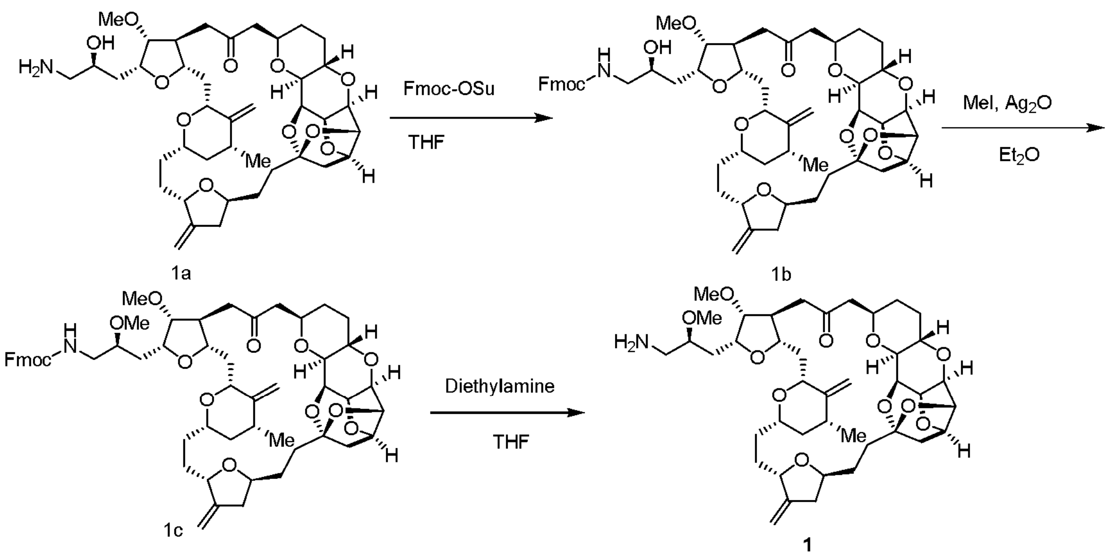


1a 1b 1c 1

Step 1: Preparation of compound **1b**

Compound **1a** (eribulin, prepared by referring to the method described in ZL201010236637.2) (72.91 mg, 0.1 mmol) was dissolved in 10 mL of tetrahydrofuran in an ice-water bath, followed by addition of Fmoc-OSu (N-(9-fluorenylmethoxycarbonyloxy)succinimide, 41 mg, 0.12 mmol). The reaction system was stirred at room temperature until the reaction was completed. The reaction system was concentrated under reduced pressure to give a crude product, which was used directly in the next step.

Step 2: Preparation of compound **1c**

The crude product of compound **1b** obtained in the previous step was dissolved in 10 mL of anhydrous ether, followed by addition of silver oxide (34.8 mg, 0.15 mmol) and iodomethane (28.4 mg, 0.2 mmol). The reaction system was stirred at room temperature until the reaction was completed. The reaction system was filtered and concentrated under reduced pressure to give a crude product, which was used directly in the next step.

Step 3: Preparation of compound **1**

The crude product of compound **1c** obtained in the previous step was dissolved in 10 mL of tetrahydrofuran, followed by addition of 2 mL of diethylamine. The reaction system was stirred at room temperature until the reaction was completed. The reaction system was concentrated under reduced pressure to obtain a crude product, which was purified by silica gel column chromatography (eluent: dichloromethane/ethyl acetate/petroleum ether) to give 3 mg of the target product, compound **1.**

LC/MS (ESI): m/z 744.2 $[M+H]^+$.

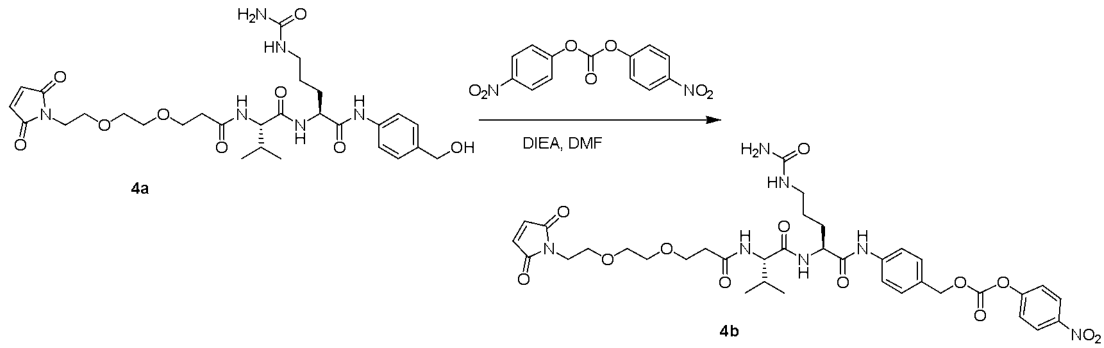


4a

4b

**4a** (50 mg, 0.08 mmol, prepared by referring to the method described in WO2017151979) was dissolved in 1.5 mL of N,N-dimethylformamide in an ice-water bath, followed by addition of DIPEA (N,N-diisopropylethylamine, 18 mg, 0.14 mmol) and bis(p-nitrophenyl)carbonate (49 mg, 0.16 mmol). The reaction system was stirred at room temperature, and 20 mL of methyl tert-butyl ether was added. The reaction system was then stirred for 20 min, filtered, and dried to give 36 mg of a solid. LC/MS (ESI): m/z 784.1 $[M+H]^+$.

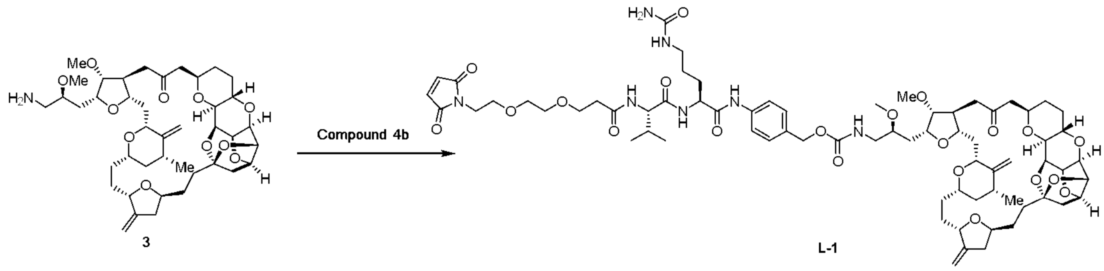


3

L-1

Compound **1** (13.5 mg, 0.018 mmol) was dissolved in 1.5 mL of DMF, followed by addition of DIPEA (7 mg, 0.054 mmol) and addition of compound **4b** (18 mg, 1.3 mmol) in portions. The reaction system was stirred until the reaction was substantially completed and then concentrated to give a crude product, which was separated by preparative HPLC to give 6.5 mg of compound L-1 (96.95% purity). LC/MS (ESI): m/z 1388.3 $[M+H]^+$.

**Example 2. ADC-001**

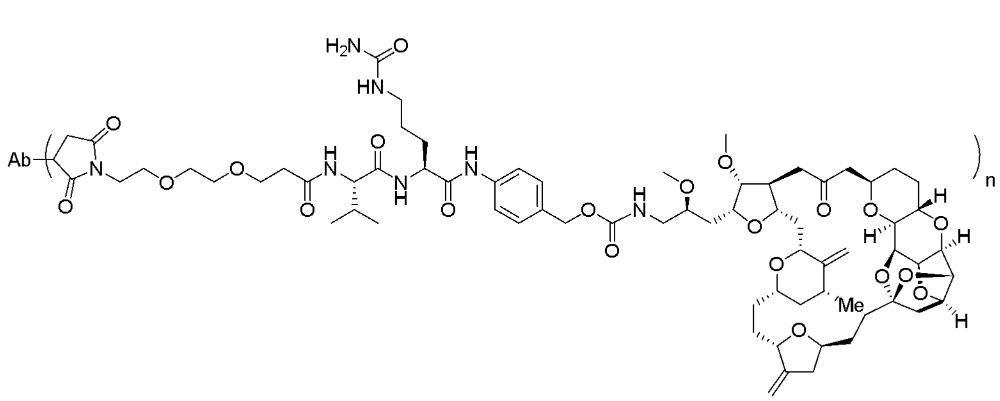

To an aqueous buffer solution of antibody pertuzumab in PBS (0.05 M aqueous PBS buffer solution at pH 6.5; 10.0 mg/mL, 1.43 mL, 97 nmol) was added a prepared aqueous solution of tris(2-carboxyethyl)phosphine hydrochloride (TCEP-HCl) (10 mM, 24.2 μL, 242 nmol) at 37 °C. The reaction system was shaken on a water bath shaker at 37 °C for 3 h, and then the reaction was stopped. The reaction solution was cooled to 25 °C in a water bath.
Compound **L-1** (1.34 mg, 965 nmol) was dissolved in 60 μL of dimethyl sulfoxide, and the mixture was added to the reaction solution described above. The resulting reaction system was shaken on a water bath shaker at 25 °C for 3 h, and then the reaction was stopped. The reaction solution was desalted and purified through a Sephadex G25 gel column (elution phase: 0.05 M aqueous PBS buffer solution at pH 6.5, containing 0.001 M EDTA) to give the title product in PBS buffer (1.24 mg/mL, 9.8 mL), which was refrigerated at 4 °C.
Mean calculated by CE-SDS: n = 3.17.

**Example 3. ADC-001**

135.32 g of antibody pertuzumab (0.93 mmol) and 0.5880 g of tris(2-carboxyethyl)phosphine hydrochloride (Sigma, 2.05 mmol) were stirred in a 20 mM histidine-hydrochloric acid-Tris buffer (pH 7.2, 7.73 L) containing 2.5 mM EDTA in a thermostatic water bath at 12 °C for 120 min to give a solution of intermediate I. 2 M acetic acid was added dropwise to adjust the pH of the reaction solution to 5.2.
Compound **L-1** (6.865 g, 4.94 mmol) was dissolved in 0.425 L of DMSO to give a solution of compound L-1 in DMSO. 0.348 L of DMSO was added to the above solution of intermediate I in advance, and the above solution of compound L-1 in DMSO was added to the solution of intermediate I to which DMSO had been added in advance. The mixture was stirred in a water bath at 12 °C. 2 M acetic acid was added dropwise to adjust the pH of the reaction solution to 4.2.
The reaction solution described above was filtered through a 0.22 μm filter and then ultrafiltered with a succinic acid buffer (pH = 4.2) to give product ADC-001 (20 g/L, average calculated by reversed-phase chromatography: n = 4.1).

**II. Biological Assays**

**Test Example 1. Inhibition of Proliferation of Human Breast Cancer BT-474 and Human Gastric Cancer NCI-N87, NCI-N87/16-8, and NCI-N87/8-2 Cells Cultured *In Vitro***

1.1 Drug information

| Sequence | Code for drug | Concentration% | Purity% |
|---|---|---|---|
| 1 | ADC-001 | 1.24 | 97.57 |
| 2 | kadcyla | 10 | / |
| Note: the above samples were provided by Shanghai Hengrui Pharmaceutical Co., Ltd., wherein the pertuzumab was prepared by a method similar to that in WO2006033700; kadcyla: trastuzumab-maitansine conjugate. | | | |

1.2 Test cells

NCI-N87 and BT-474 cells were purchased from American Type Culture Collection (ATCC). T-DM1-resistant NCI-N87/16-8 and NCI-N87/8-2 cells induced by long-term effect of T-DM1 on NCI-N87 were constructed in this laboratory. Cells were cultured in an RPMI 1640/DMEM (1:1) medium containing 10% fetal bovine serum (FBS).

1.3 Instruments and reagents

RPMI 1640 and DMEM were purchased from Gibco BRL; FBS was purchased from Gibco; sulforhodamine B (SRB) was purchased from Sigma.
Microplate reader Synergy H4 was purchased from BioTek.

1.4 Experimental procedures

A certain number of cells in logarithmic growth phase were seeded into a 96-well culture plate. After the cells were subjected to adherence culture for 24 h, the drug at different concentrations (10000 ng/mL, 3000 ng/mL, 1000 ng/mL, 300

ng/mL, 100 ng/mL, 30 ng/mL, 10 ng/mL, 3 ng/mL, and 1 ng/mL) was added. After 120 h of drug treatment, the cells were immobilized with trichloroacetic acid. Then the cells were stained with an SRB solution; finally, a Tris solution was added to dissolve SRB. OD values at the wavelength of 510 nm were read using a microplate reader, and the cell growth inhibition rate was calculated according to the following formula:

Inhibition rate = (OD value of control well - OD value of dosing well)/OD value of control well × 100%

Half maximal inhibitory concentrations ($IC_{50}$ values) were calculated from inhibition rates at each concentration using GraphPad Prism 7 software.

Table 1. Inhibition ($IC_{50}$) of proliferation of HER2-positive tumor cells (ng/mL)

| | NCI-N87 | NCI-N87/16-8 | NCI-N87/8-2 | BT-474 |
|---|---|---|---|---|
| ADC-001 | 8.1 | 33.6 | 13.0 | 5.6 |

**Test Example 2. *In Vitro* Cytotoxic Activity Screening of Compound 1**

1.1. Principle and method

In this experiment, the ATP content was determined using CTG to reflect the survival condition of the tumor cells. First, cells were seeded at different densities and cultured for 3 days and 5 days, and then the final culture condition was determined based on the $IC_{50}$ and the maximum inhibition rate. The killing effect of the toxin molecule was then assayed according to this condition.

1.2. Selection of cell strains

According to the purpose of the experiment, two disease models of breast cancer and NSCLC were selected, and three cell strains of SKBR3 tumor cells (HER2+, ATCC, Cat# HTB-30), MDA-MB-468 (HER2-, ATCC, Cat# HTB-132) and A549 (human non-small cell lung cancer cells, ATCC, Cat# CCL-185) were selected for screening.

1.3. Determination of cell culture conditions

1) Cell culturing: A549, SK-BR-3 and MDA-MB-468 cells were cultured in Ham's F-12K (Kaighn's) medium (Gibco, 21127030), McCoy's 5A medium (ThermoFisher, Cat# 16600108) and Leibovitz's L-15 medium (ThermoFisher, Cat# 11415-114) containing 10% FBS (Gibco, 10099-141), respectively.
2) Cell plating: A549 cells were digested with pancreatin, then the digestion was terminated with the above medium. The cells were counted, and $4.3 \times 10^5$, $7.2 \times 10^5$ and $11.5 \times 10^5$ cells were each added to a culture solution to give a final volume of 26 mL. 180 μL of cell suspension was added to each well in columns 2 to 11 of a 96-well plate (Corning, Cat# 3903) to give cell densities of 3K, 5K, and 8K per well. Wells in column 12 were each filled with 200 μL of culture solution and the remaining wells were each filled with PBS. The SKBR3 and MDA-MB-468 cells were subjected to the same operations described above. Each sample was run in duplicate.
3) Drug preparation: stock solutions of positive control eribulin and the compound of the present disclosure were prepared in DMSO in a 96-well round-bottom plate (Corning, Cat# 3788). 2 mM stock solution (stock solution diluted 10-fold in DMSO) was prepared in column 1 of drug preparation plate 1, and then 10-fold gradient dilution in DMSO was performed through column 10, and the wells in column 11 were filled with DMSO. 95 μL of corresponding culture solution was added into each well from column 2 to column 11 of the drug preparation plate 2, and 5 μL of solution was pipetted from column 2 to column 11 of drug preparation plate 1 to drug preparation plate 2. The solution was mixed well, and 20 μL of solution was pipetted and added into the plated cells. The cells were then cultured for 3 days and 5 days.
4) CTG assay (Cell Titer-GloTM, luminescent cell viability assay, Promega): the cell plates were taken out on day 3 and day 5 and equilibrated to room temperature. 90 μL of CTG was added into each well, and the mixture was reacted at room temperature for 10 min in the dark. The luminescence value was read using a microplate reader and $IC_{50}$ was calculated.

1.4. Data results

Table 2

| | SKBR3 | | MDA-MB-468 | | A549 | |
|---|---|---|---|---|---|---|
| Compound | $IC_{50}$ (nM) | Maximum inhibition rate (%) | $IC_{50}$ (nM) | Maximum inhibition rate (%) | $IC_{50}$ (nM) | Maximum inhibition rate (%) |
| Eribulin | 0.7147 | 94.90 | 0.4819 | 87.47 | 0.6609 | 81.50 |
| Compound 1 | 0.2052 | 96.87 | 0.1827 | 88.01 | 0.5151 | 81.34 |

**Test Example 3. Pharmacokinetic Study of Compound 1**

1. Overview

Non-naive beagles were taken as test animals, and the drug concentrations in plasma at various times after intravenous administration of beagles with compound 1 and eribulin were measured by using LC/MS/MS. The pharmacokinetic behavior of the compound of the present disclosure in dogs was studied, and its pharmacokinetic profile was evaluated.

2. Method

2.1. Test compounds

Compound 1 and eribulin

2.2. Test animals

Six male beagles were divided into 2 groups of 3, purchased from Shanghai Medicilon Inc., and subjected to an administration test.

2.3. Compound solution preparation

Compound 1 was weighed out and dissolved in 5% (v/v) of DMSO, 20% (v/v) of PG, and 20% (v/v) of PEG400, and then 55% (v/v) of normal saline was added to obtain a 0.25 mg/mL colorless and clear solution.
Eribulin was weighed out and dissolved in 5% (v/v) of DMSO, 20% (v/v) of PG and 20% (v/v) of PEG400, and then 55% (v/v) of normal saline was added to obtain a 0.25 mg/mL colorless and clear solution.

2.4. Administration

Beagles in one group were intravenously injected with compound 1 at a dose of 0.5 mg/kg and at a volume of 2 mL/kg.
Beagles in another group were intravenously injected with eribulin at a dose of 0.5 mg/kg and at a volume of 2 mL/kg.

3. Procedure

The beagles were injected with compound 1, and 1 mL of blood was collected before administration and at 5 min, 0.25 h, 0.5 h, 1.0 h, 2.0 h, 4.0 h, 8.0 h, 12.0 h, and 24.0 h after administration. The collected blood samples were each placed in EDTA-K2 anticoagulant blood collection tubes, and the collected whole blood was placed on ice and centrifuged within 1 h to isolate the plasma (centrifugal force: 2200 g, centrifugal time: 10 min, 2-8 °C). Plasma samples were stored in a refrigerator at -80 °C prior to testing.
The beagles were injected with compound eribulin, and 1 mL of blood was collected before administration and at 5 min, 0.25 h, 0.5 h, 1.0 h, 2.0 h, 4.0 h, 8.0 h, 12.0 h, and 24.0 h after administration. The collected blood samples were each placed in EDTA-K2 anticoagulant blood collection tubes, and the collected whole blood was placed on ice and centrifuged within 1 h to isolate the plasma (centrifugal force: 2200 g, centrifugal time: 10 min, 2-8 °C). Plasma samples were stored in a

refrigerator at -80 °C prior to testing.

The content of the test compounds in beagle plasma after the injection was determined: 25 μL of beagle plasma at each time point after the administration was mixed with 50 μL (100 ng/mL) of internal standard solution camptothecin (China National Institutes for Food and Drug Control) and 200 μL of acetonitrile. The mixture was vortexed for 5 min and centrifuged for 10 min (3700 rpm), and 3-4 μL of the supernatant of the plasma sample was taken for LC/MS/MS assay (API4000 triple quadrupole tandem mass spectrometer (No. 2), Applied Biosystems, USA; Shimadzu, LC-30AD ultra high performance liquid chromatography system, Shimadzu, Japan).

4. Pharmacokinetic parameters

Pharmacokinetic parameters for the compound of the present disclosure are shown in Table 3 below.

Table 3

| No. | Pharmacokinetic experiment for beagles | | | | | |
|---|---|---|---|---|---|---|
| | Plasma concentration | Area under curve | Half-life | Retention Time | Clearance rate | Apparent volume of distribution |
| | C5min (ng /mL) | AUC0-t (ng /mL*h) | T1/2 (h) | MRT (h) | CL (ml/min/kg) | Vz (ml/kg) |
| Compound 1 | 82.1 | 433 | 29.3 | 41.1 | 8.9 | 22039 |
| Eribulin | 217 | 106 | 3.5 | 3.7 | 78 | 15556 |

**Test Example 4. Therapeutic Effect of ADC-001 on Human Gastric Cancer NCI-N87/16-8 Nude Mouse Subcutaneous Xenograft Tumor**

1. Objective

The objective is to evaluate the therapeutic effect of ADC-001 on human gastric cancer NCI-N87/16-8 nude mouse subcutaneous xenograft tumor.

2. Test compound

ADC-001, diluted to the desired concentration with normal saline.

3. Cells

Human gastric cancer NCI-N87 cells were purchased from American Type Culture Collection. NCI-N87 was subjected to long-term induction with T-DM1 to develop resistance to T-DM1 and was named NCI-N87/16-8. The cells were cultured in a 10 cm petri dish with an RPMI 1640 medium (Gibco) containing 10% fetal bovine serum, penicillin, and streptomycin in a 5% $CO_2$ incubator at 37 °C. Subculturing was performed 2-3 times a week. When the cells were in exponential growth phase, they were digested with pancreatin, collected, counted and inoculated.

4. Test animals

BALB/cJGpt-Foxn1$^{nu}$/Gpt mice, aged 4 weeks, female, purchased from Jiangsu Gem-Pharmatech Co., Ltd. Production license No.: SCXK (Jiangsu) 2019-0009; animal certification No.: 202004958. Housing environment: SPF.

5. Test procedures

Each nude mouse was inoculated subcutaneously with 1 × $10^7$ NCI-N87/16-8 cells, and after tumors grew to 100-150 $mm^3$, the animals were grouped according to tumor volume and body weight (D0). The mice were intravenously injected with 0.3 mg/mL and 0.6 mg/mL of ADC-001 at a volume of 10 mL/kg. The tumor volume and body weight were measured twice a week, and data were recorded.

6. Test indexes

The experimental index was to investigate the influence of the drug on the tumor growth, and the specific index was T/C% or tumor growth inhibition (TGI%).

Tumor diameters were measured twice a week with a vernier caliper and tumor volume (V) was calculated according to the following formula:

$V = 1/2 \times a \times b^2$ where a and b represent length and width, respectively.

T/C (%) = $(T - T_0)/(C - C_0) \times 100$, where T and C represent the tumor volumes of animals at the end of the experiment; $T_0$ and $C_0$ represent the tumor volumes of animals at the beginning of the experiment.

$$\text{Tumor growth inhibition \% (TGI\%)} = 100 - \text{T/C (\%)}.$$

When the tumor started to regress, tumor growth inhibition % (TGI%) = $100 - (T - T_0)/T_0 \times 100$.

If the tumor shrinks compared to its initial volume, i.e., $T < T_0$ or $C < C_0$, it is defined as partial regression (PR) of the tumor. If the tumor completely disappears, it is defined as complete regression (CR) of the tumor.

At the end of the experiment, at the experiment endpoint, or when the mean tumor volume in the solvent group reached 1500 $mm^3$, the animals were sacrificed by $CO_2$ anesthesia and dissected to give the tumors. The tumors were photographed.

7. Statistical analysis

Unless otherwise indicated, comparison between tumor volumes of the two groups was made by two-tailed Student's t-test, with $P < 0.05$ defined as a statistically significant difference.

8. Results

On day 22 after a single administration at doses of 3 mg/kg and 6 mg/kg, ADC-001 could inhibit the growth of NCI-N87/16-8 cells in the nude mouse subcutaneous xenograft tumor model, and the tumor growth inhibition rates were 46.77% and 90.18%, respectively ($P < 0.01$ vs PBS control). The tumor-bearing mice were able to well tolerate ADC-001, and symptoms such as weight loss were not found.

9. Conclusion

ADC-001 can effectively inhibit the growth of human gastric cancer NCI-N87/16-8 cells in a nude mouse subcutaneous xenograft tumor model, and tumor-bearing mice can well tolerate ADC-001.

**Test Example 5. Therapeutic Effect of ADC-001 on JIMIT-1 Nude Mouse Subcutaneous Xenograft Tumor**

1. Test compound

ADC-001, diluted to the desired concentration with PBS.

2. Cells

Human breast cancer JIMIT-1 cells.

3. Test animals

Female Balb/c nude mice

4. Test procedures

BALB/c nude mice were each inoculated subcutaneously in the right dorsal part with JIMT1 cells $5 \times 10^5$ JIMT1 cells. When the tumor volume reached 80-100 $mm^3$, the mice were randomly divided into groups of 6 according to the tumor volume and body weight, and the administration was started on the day of the grouping. HRA00092-C063-004 was diluted to 0.6 mg/mL with PBS and injected intravenously at a volume of 10 mL/kg, and the mice in the control group were injected intravenously with PBS at the same volume.

The long and short diameters of the tumors were measured twice a week with a vernier caliper and the body weight of the mice was also measured.
Tumor volume (V) was calculated according to the following formula:
$V = 1/2 \times a \times b^2$ where a and b represent length and width, respectively.
T/C (%) = $(T - T_0)/(C - C_0) \times 100$, where T and C represent the tumor volumes of animals at the end of the experiment; $T_0$ and $C_0$ represent the tumor volumes of animals at the beginning of the experiment.

$$\text{Tumor growth inhibition \% (TGI\%)} = 100 - \text{T/C (\%)}.$$

Unless otherwise indicated, comparison between tumor volumes of the two groups was made by two-tailed Student's t-test, with $P < 0.05$ defined as a statistically significant difference.

5. Results

On day 24 after a single administration, ADC-001 could significantly inhibit the growth of JIMIT-1 cells in the nude mouse subcutaneous xenograft tumor model, and the tumor growth inhibition rate was 121.3%, and tumor regression was found in 5 mice (< 50 $mm^3$). The tumor-bearing mice were able to well tolerate ADC-001, and symptoms such as weight loss were not found.

**III. Formulations**

**The equipment used in the formulation preparation and determination and the calculation method for results are shown below:**

**SEC molecular exclusion chromatography:**

This is an analytical method that separates a solute based on the relative relationship between the pore size of the gel pores and the coil size of the polymer sample molecule. SEC% (SEC monomer content percentage) = A monomer/A total $\times$ 100% (A monomer represents the peak area of the main peak monomer in the sample, and A total represents the sum of all peak areas). ΔSEC% = SEC% of formulation before stability storage - SEC% of formulation after stability storage.
Instrument for SEC determination: Agilent 1260; column: waters, XBridge BEH200Å SEC (300 $\times$ 7.8 mm 3.5 μm).

**R-CE capillary gel electrophoresis:**

This is a type of electrophoresis in which a gel is moved into a capillary as a support medium and a method that achieves separation under a certain voltage based on the molecular weight of the sample.
R-CE% = A main peak/A total $\times$ 100% (A main peak represents the light chain main peak area + the heavy chain main peak area, and A total represents the sum of all peak areas). ΔR-CE% = R-CE% of formulation before stability storage - R-CE% of formulation after stability storage.
Instrument for CE determination: Beckman model plus800

**Osmotic pressure determination:**

The freezing point method is used for measuring osmotic pressure. On the basis of the directly proportional relationship between the freezing point depression value and the molar concentration of a solution, the freezing point of the solution is determined using a highly sensitive temperature-sensing element and then converted into the osmotic pressure through electric quantity. Manufacturer of instrument: Loser, model: OM815.

**Protein concentration:**

The protein used in the following examples was anti-Her2-ADC.
Instrument for protein concentration determination: ultraviolet-visible spectrophotometer; model: Nano Drop 2000; optical path length: 1 mm.
In the present disclosure, the concentration of the antibody-drug conjugate is expressed based on the concentration of the protein, i.e., the concentration of the antibody moiety in the antibody-drug conjugate.

Since the toxin in the antibody-drug conjugate shows almost no absorption both at the characteristic absorption wavelength of the protein of 280 nm and at 370 nm, the following formula was used to calculate the above protein concentration:

$$A_{280\ nm} = (C_{mAb} \times E_{mAb-280}) \times l$$

Namely:

$$C_{mAb} = \frac{A_{280\ nm}}{E_{mAb-280}}$$

In the formula, $A_{280\ nm}$: the average absorbance of a single sample of the test sample solution at an optical path length of 1 cm and a wavelength of 280 nm;
$E_{mAb-280}$: the mass extinction coefficient of the protein at the wavelength of 280 nm, which is 1.45 $g^{-1}cm^{-1}L$;
$C_{mAb}$: the concentration of the protein, mg/mL;
l: the optical path length, cm (here the optical path length is 1 cm).

If the test solution is diluted, the protein concentration is: C (mg/mL) = $C_{mAb} \times$ N, and N is the dilution factor.

**Free toxin determination (RP-UPLC reversed-phase method):**

The protein in the sample was precipitated and removed by using ACN, and the supernatant was taken, dried with nitrogen, and then reconstituted. The reconstituted solution was separated according to different polarities of toxins in the sample, and then the toxin content in the sample was calculated by a linear equation for fitting the toxin standard concentration-peak area.
Instrument for determination: waters ACQuity H class.
Chromatographic column: waters ACQUITY UPLC BEH Shield RP18, 1.7 μm, 2.1 × 150 mm.

**Free toxin determination (LC-MS method):**

In this assay, the toxins and related impurities in the ADC product were quantitatively determined by liquid chromatography-mass spectrometry. In this method, the sample was directly diluted and loaded onto the UPLC-MS system for detection. In addition, the free toxin content in the test sample was determined by the external standard curve method combined with the extracted ion chromatographic peak area of the toxin standard.
Instrument: Waters, Acquity UPLC_Rda (Bioaccord).
Column: Waters, ACQUITY UPLC BEH C4, 1.7 μm, 2.1 × 50 mm.

**Formulation Example 1. pH and Buffer System Screening**

Formulations containing the buffer systems shown in Table 4, 20 mg/mL protein, 0.2 mg/mL polysorbate 80 (PS80), 40 mg/mL sucrose, and 9 mg/mL glycine were prepared. Samples were subjected to a forced degradation study (storage at 40 °C for one month), and the effects of different buffer systems on protein stability were evaluated based on SEC.
The results are shown in Table 4. The SEC data show that after storage at 40 °C for one month, the formulation containing His-HCl or SA showed a smaller decrease in SEC monomer than the CA group. The His-HCl and SA systems were preferred.

Table 4. Results of pH and buffer system screening

| Group | Buffer system | Storage conditions | △SEC% |
|---|---|---|---|
| 1 | 30 mM His-HCl pH 5.5 | 40 °C M1 | 23.6 |
| 2 | 30 mM SA pH 5.5 | 40 °C M1 | 27.5 |

(continued)

| Group | Buffer system | Storage conditions | △SEC% |
|---|---|---|---|
| 3 | 30 mM CA pH 5.5 | 40 °C M1 | 30.1 |
| Note: His-HCl represents histidine-histidine hydrochloride; CA represents citric acid-sodium citrate; SA represents succinic acid-sodium succinate; 40 °C M1: storage at 40 °C for one month; △SEC%: SEC monomer difference between D0 and 40 °C M1; the same applies below. | | | |

**Formulation Example 2. Effect of pH of Buffer System on Free Toxin**

Formulations containing the buffer systems shown in Table 5, 20 mg/mL protein, 0.2 mg/mL PS80, 40 mg/mL sucrose, and 9 mg/mL glycine were prepared. Samples were subjected to a stability study at 2-8 °C, and the effects of buffer systems at different pH values on the free toxin were evaluated based on free toxin.

The results are shown in Table 5. The free toxin data show that after storage at 2-8 °C for one month, the free toxin decreased as the pH in the buffer system decreased. pH 5.5 was superior to pH 6.5.

Table 5. Effect of pH on free toxin

| Group | Buffer system | Storage conditions | Free toxin-compound 3 (ppm) |
|---|---|---|---|
| 1 | 30 mM His-HCl pH 6.5 | 2-8 °C M1 | 2296 |
| 2 | 30 mM His-HCl pH 5.5 | 2-8 °C M1 | 137 |
| Note: 2-8 °C M1 represents storage at 2-8 °C for one month. | | | |

**Formulation Example 3. Effect of pH and Buffer System on Free Toxin**

Formulations containing the buffer systems shown in Table 6, 20 mg/mL protein, 0.2 mg/mL polysorbate 80 (PS80), 40 mg/mL sucrose, and 9 mg/mL glycine were prepared. Samples were subjected to a stability study (storage at room temperature for one day, storage at 2-8 °C for one day, and 2 repeated freeze-thaw cycles between -35 °C and 2-8 °C), and the effects of different pH values and buffer systems on the free toxin were evaluated based on free toxin.

The results are shown in Table 6. The free toxin data show that as the pH decreased, the increase speed of the free toxin slowed down, and the buffer system SA was superior to His-AA. Thus, the buffer system was preferably 30 mM SA pH 4.2.

Table 6. Results of effect of pH and buffer systems on free toxin

| Group | Buffer system | Storage conditions | △Free toxin-compound 3 |
|---|---|---|---|
| 1 | 30 mM His-AA pH 4.8 | RT24H | 247.2 |
| | | 2-8 °C D1 | 113.3 |
| | | FT2C | 25.4 |
| 2 | 30 mM SA pH 4.8 | RT24H | 84.0 |
| | | 2-8 °C D1 | 23.3 |
| | | FT2C | 19.8 |
| 3 | 30 mM SA pH 4.2 | RT24H | 6.0 |
| | | 2-8 °C D1 | 1.7 |
| | | FT2C | 2.2 |
| Note: RT24H represents storage at room temperature for one day; 2-8 °C D1 represents storage at 2-8 °C for one day; FT2C represents 2 repeated freeze-thaw cycles between -35 °C and 2-8 °C; △free toxin represents an increase in free toxin compared to time 0 after storage; the same applies below. | | | |

**Formulation Example 4. Optimization of pH of Buffer System**

Solutions and freeze-dried powder formulations containing the buffer systems shown in Table 7, 20 mg/mL protein, 0.6 mg/mL poloxamer 188 (F68), 40 mg/mL sucrose, and 9 mg/mL glycine were prepared. Samples were

subjected to a stability study (solution samples were left to stand at room temperature for 24 h, left to stand at 2-8 °C for 3 days, and subjected to 5 repeated freeze-thaw cycles between -35 °C and 2-8 °C, and freeze-dried powder samples were left to stand at 40 °C for 4 weeks). The effects of buffer systems at different pH values on protein stability and free toxin were evaluated based on appearance, SEC, R-CE, and free toxin.

The results are shown in Tables 7 and 8. The results show that the solutions exhibited relatively good SEC/CE/free toxin stability under various conditions at pH 4.0-4.2; and showed slightly increased free toxin amount under the conditions of RT24h and 2-8 °C D3 at pH 4.6, but the overall free toxin amount was still relatively low, and the stability was acceptable. The freeze-dried formulations exhibited relatively good stability at pH 4.0-4.6 after storage at 40 °C for 4 weeks, with no difference among the groups. Based on the solution and lyophilization results, the buffer system was preferably 30 mM SA pH 4.2.

Table 7. Results of optimized solutions for pH of buffer systems

| Buffer system, pH | Storage conditions | SEC% | R-CE% | Free toxin |
|---|---|---|---|---|
| | | Monomer | | Compound 3 |
| 30 mM SA pH 4.0 | D0 | 96.8 | 98.0 | <50 |
| | RT24H | 96.9 | 97.4 | <50 |
| | 2-8 °C D3 | 96.9 | 98.0 | <50 |
| | FT5C | 96.9 | 98.4 | <50 |
| 30 mM SA pH 4.2 | D0 | 96.6 | 98.1 | <50 |
| | RT24H | 96.7 | 98.2 | 58.0 |
| | 2-8 °C D3 | 96.7 | 98.2 | <50 |
| | FT5C | 96.7 | 98.3 | <50 |
| 30 mM SA pH 4.6 | D0 | 96.4 | 97.9 | <50 |
| | RT24H | 96.4 | 98.0 | 89.2 |
| | 2-8 °C D3 | 96.5 | 97.9 | 60.1 |
| | FT5C | 96.4 | 98.3 | 54.7 |
| Note: D0 represents day 0; RT24H represents room temperature for 24 h; D3 represents 3 days, and so on; FT1C represents 1 repeated freeze-thaw cycle between -35 °C and 2-8 °C, and so on. | | | | |

Table 8. Results of optimized freeze-dried powders for pH of buffer systems

| Buffer system, pH | Storage conditions | Appearance | SEC% | R-CE % | Free toxin |
|---|---|---|---|---|---|
| | | | Monomer | | Compound 3 |
| 30 mM SA pH 4.0 | D0 | Clear and transparent | 96.8 | 98.0 | <50 |
| | FR-40 °C W4 | Clear and transparent | 96.1 | 98.3 | <50 |
| 30 mM SA pH 4.2 | D0 | Clear and transparent | 96.6 | 98.1 | <50 |
| | FR-40 °C W4 | Clear and transparent | 95.9 | 98.1 | <50 |
| 30 mM SA pH 4.6 | D0 | Clear and transparent | 96.4 | 97.9 | <50 |
| | FR-40 °C W4 | Clear and transparent | 96.0 | 98.0 | <50 |
| Note: FR represents that the freeze-dried powder was reconstituted with water for injection; D0 represents day 0; W4 represents 4 weeks. | | | | | |

**Formulation Example 5. Excipient and Osmotic Pressure Regulator Screening**

Solutions containing the excipients and osmotic pressure regulators shown in Table 9, 30 mM SA pH 4.2, 20 mg/mL protein, and 0.2 mg/mL PS80 were prepared. Samples were subjected to a stability study, and the effects of excipients and osmotic pressure regulators at different concentrations on protein stability were evaluated based on appearance, SEC, and free toxin.

The results are shown in Table 10. The RT24H/2-8 °C D1/FT1C data show that there was no significant difference in appearance and SEC between the groups. However, the group of 40 mg/mL sucrose + 9 mg/mL glycine exhibited slightly better free toxins, and thus, 40 mg/mL sucrose and 9 mg/mL glycine were preferred.

Table 9. Information about excipients and osmotic pressure regulators

| Group | Excipient | Osmotic pressure regulator |
|---|---|---|
| Group | Excipient | Osmotic pressure regulator |
| 1 | 40 mg/mL sucrose | 9 mg/mL glycine |
| 2 | 20 mg/mL sucrose | 13.5 mg/mL glycine |

Table 10. Results of excipient and osmotic pressure regulator screening

| Group | **Storage conditions** | **Solution appearance** | ΔSEC% | ΔFree toxin-compound 3 (ppm) |
|---|---|---|---|---|
| 1 | RT24H | Clear and transparent | -0.3 | 6.0 |
| | 2-8 °C D1 | Clear and transparent | -0.7 | 1.7 |
| | FT1C | N/A | N/A | 2.6 |
| 2 | RT24H | Clear and transparent | -0.2 | 12.8 |
| | 2-8 °C D1 | Clear and transparent | -0.4 | 3.5 |
| | FT1C | N/A | N/A | 3.5 |
| Note: RT24H represents storage at room temperature for 24 h; 2-8 °C D1 represents storage at 2-8 °C for 1 day; FT1C represents 1 repeated freeze-thaw cycle between -35 °C and 2-8 °C; N/A represents no detection. | | | | |

**Formulation Example 6. Surfactant Screening**

Solutions containing the surfactants shown in Table 11, 30 mM SA pH 4.2, 20 mg/mL protein, 40 mg/mL sucrose, and 9 mg/mL glycine were prepared. Samples were left to stand for stability (storage at room temperature for 7 days and at 4 °C for 7 days), and the effects of different surfactants on protein stability were evaluated based on SEC and free toxin.

The results are shown in Table 11. The data show that there was no significant difference in free toxin and SEC purity between the groups.

Table 11. Results of surfactant type screening

| Group | Surfactant | Storage conditions | ΔSEC% | ΔFree toxin-compound 3 (ppm) |
|---|---|---|---|---|
| 1 | 0.2 mg/mL PS80 | RTD7 | 0.6 | 113 |
| | | 4 °C D7 | 0.5 | 55 |
| 2 | 0.6 mg/mL F68 | RTD7 | 0.1 | 101 |
| | | 4 °C D7 | 0.3 | 50 |

**Formulation Example 7. Surfactant Concentration Screening**

Solutions and freeze-dried powder formulations containing F68 at different concentrations shown in Table 12, 30 mM SA pH 4.2, 20 mg/mL protein, 40 mg/mL sucrose, and 9 mg/mL glycine were prepared. Samples were subjected to a stability study (solution samples were left to stand at 2-8 °C for 3 days, subjected to 3 repeated freeze-thaw cycles between -35 °C and 2-8 °C, and lyophilized to give products at time 0), and the effects of F68 at different concentrations on protein stability were evaluated based on appearance and SEC.

The results are shown in Table 12. The data show that for the solutions, 0.4-0.6 mg/mL F68 formulas exhibited relatively good sample appearance under different conditions, and there was no significant difference in SEC purity. There was no significant difference in appearance and SEC purity after lyophilization and reconstitution. Therefore, the surfactant concentration was preferably 0.4-0.6 mg/mL, more preferably 0.6 mg/mL.

Table 12. Results of surfactant concentration screening

| Group | Surfactant | Storage conditions | Appearance | ΔSEC% |
|---|---|---|---|---|
| 1 | 0.2 mg/mL F68 | Solution D0 | Occasional fine particles | N/A |
| | | 2-8 °C D3 | A small number of fine particles | 0.1 |
| | | FT3C | A small number of fine particles | 0.0 |
| | | FR-DO | Clear and transparent | N/A |
| 2 | 0.4 mg/mL F68 | Solution D0 | Clear and transparent | N/A |
| | | 2-8 °C D3 | Occasional fine particles | 0.2 |
| | | FT3C | Clear and transparent | 0.1 |
| | | FR-DO | Clear and transparent | N/A |
| 3 | 0.6 mg/mL F68 | Solution D0 | Clear and transparent | N/A |
| | | 2-8 °C D3 | Clear and transparent | 0.1 |
| | | FT3C | Clear and transparent | 0.1 |
| | | FR-DO | Clear and transparent | N/A |

## Claims

**1.** A pharmaceutical composition, comprising an antibody-drug conjugate and a buffer, wherein the antibody-drug conjugate has a structure represented by formula I:

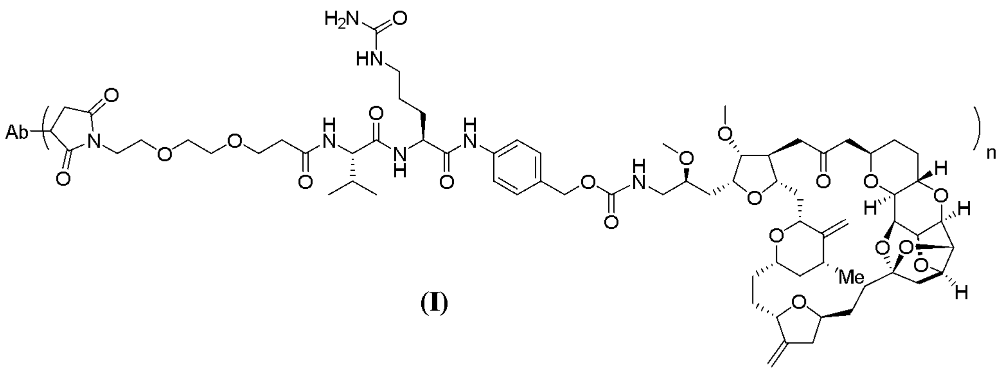

(I)

;

wherein:

Ab is pertuzumab;
n is 1 to 10, preferably 1 to 8, and more preferably 3 to 5;
the buffer is selected from the group consisting of a citrate buffer, a histidine buffer, and a succinate buffer, preferably histidine-histidine hydrochloride, citric acid-sodium citrate, and succinic acid-sodium succinate.

**2.** The pharmaceutical composition according to claim 1, wherein the pharmaceutical composition has a pH of 3.5 to 5.5, preferably a pH of 3.5 to 5.0, and more preferably a pH of 3.7 to 4.7.

**3.** The pharmaceutical composition according to claim 1, wherein the buffer is at a concentration of 5 mM to 50 mM, preferably 20 mM to 40 mM, and more preferably 30 mM.

**4.** The pharmaceutical composition according to claim 1 or 2, wherein the pharmaceutical composition further comprises a surfactant, and the surfactant is preferably polysorbate or poloxamer.

**5.** The pharmaceutical composition according to claim 4, wherein the surfactant is at a concentration of 0.01 mg/mL to 1.0 mg/mL, preferably 0.05 mg/mL to 1.0 mg/mL, and more preferably 0.1 mg/mL to 1.0 mg/mL.

6. The pharmaceutical composition according to any one of claims 1 to 5, wherein the pharmaceutical composition further comprises a sugar, and the sugar is preferably sucrose.

7. The pharmaceutical composition according to claim 6, wherein the sugar is at a concentration of 25 mg/mL to 80 mg/mL, preferably 30 mg/mL to 50 mg/mL, and more preferably 40 mg/mL.

8. The pharmaceutical composition according to any one of claims 1 to 7, wherein the pharmaceutical composition further comprises an amino acid, and the amino acid is preferably glycine.

9. The pharmaceutical composition according to claim 8, wherein the amino acid is at a concentration of 6 mg/mL to 15 mg/mL, preferably 7 mg/mL to 11 mg/mL, and more preferably 9 mg/mL.

10. The pharmaceutical composition according to any one of claims 1 to 9, wherein the antibody-drug conjugate is at a concentration of 1 mg/mL to 100 mg/mL on a protein concentration basis;

    preferably, the antibody-drug conjugate is at a concentration of 10 mg/mL to 30 mg/mL on a protein concentration basis;
    more preferably, the antibody-drug conjugate is at a concentration of 18 mg/mL to 22 mg/mL on a protein concentration basis.

11. The pharmaceutical composition according to any one of claims 1 to 10, comprising: (a) on a protein concentration basis, 1 mg/mL to 100 mg/mL said antibody-drug conjugate, (b) 0.1 mg/mL to 0.8 mg/mL poloxamer or polysorbate, (c) 30 mg/mL to 50 mg/mL sucrose, (d) 6 mg/mL to 15 mg/mL glycine, and (e) 10 mM to 50 mM succinic acid-sodium succinate buffer; the pharmaceutical composition having a pH of 3.5 to 5.5, wherein preferably, the pharmaceutical composition comprises:

    (a) on a protein concentration basis, 1 mg/mL to 50 mg/mL said antibody-drug conjugate, (b) 0.4 mg/mL to 0.8 mg/mL poloxamer or polysorbate, (c) 30 mg/mL to 50 mg/mL sucrose, (d) 6 mg/mL to 15 mg/mL glycine, and (e) 20 mM to 40 mM succinic acid-sodium succinate buffer; the pharmaceutical composition having a pH of 3.7 to 4.7;
    more preferably, the pharmaceutical composition comprises:
    (a) on a protein concentration basis, 20 mg/mL said antibody-drug conjugate, (b) 0.6 mg/mL poloxamer 188 or 0.2 mg/mL polysorbate 80, (c) 40 mg/mL sucrose, (d) 9 mg/mL glycine, and (e) 30 mM succinic acid-sodium succinate buffer; the pharmaceutical composition having a pH of 4.2.

12. A freeze-dried formulation comprising an antibody-drug conjugate, wherein the freeze-dried formulation is capable of forming the pharmaceutical composition according to any one of claims 1 to 11 after being reconstituted.

13. A freeze-dried formulation comprising an antibody-drug conjugate, wherein the freeze-dried formulation is obtained by lyophilizing the pharmaceutical composition according to any one of claims 1 to 11.

14. A reconstituted solution comprising an antibody-drug conjugate, wherein the reconstituted solution is prepared by reconstituting the freeze-dried formulation according to claim 12 or 13.

15. A product, comprising a container, wherein the container contains the pharmaceutical composition according to any one of claims 1 to 11, the freeze-dried formulation according to claim 12 or 13, or the reconstituted solution according to claim 14.

16. Use of the pharmaceutical composition according to any one of claims 1 to 11, the freeze-dried formulation according to claim 12 or 13, the reconstituted solution according to claim 14, or the product according to claim 15 in the manufacture of a medicament for treating a viral infection, a tumor, or a cancer, wherein preferably, the tumor is a cancer associated with expression of a domain II of HER2; preferably, the tumor is selected from the group consisting of breast cancer, ovarian cancer, cervical cancer, uterine cancer, prostate cancer, renal cancer, urethral cancer, bladder cancer, liver cancer, gastric cancer, endometrial cancer, salivary gland cancer, esophageal cancer, melanoma, neuroglioma, neuroblastoma, sarcoma, lung cancer, colon cancer, rectal cancer, colorectal cancer, leukemia, bone cancer, skin cancer, thyroid cancer, pancreatic cancer, and lymphoma.

N87/16-8 subcutaneous xenograft tumor model-tumor volume

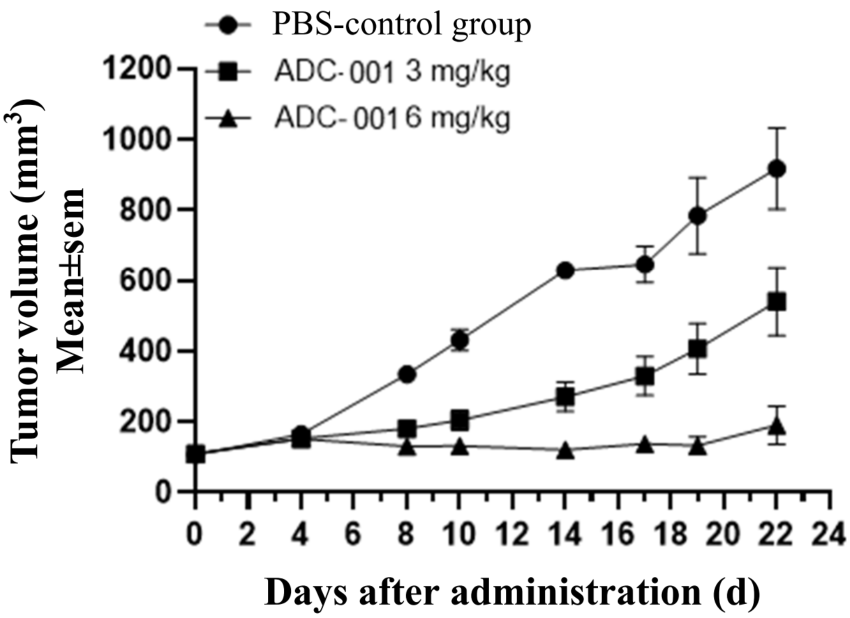


FIG. 1

N87/16-8 subcutaneous xenograft tumor model-body weight of mouse

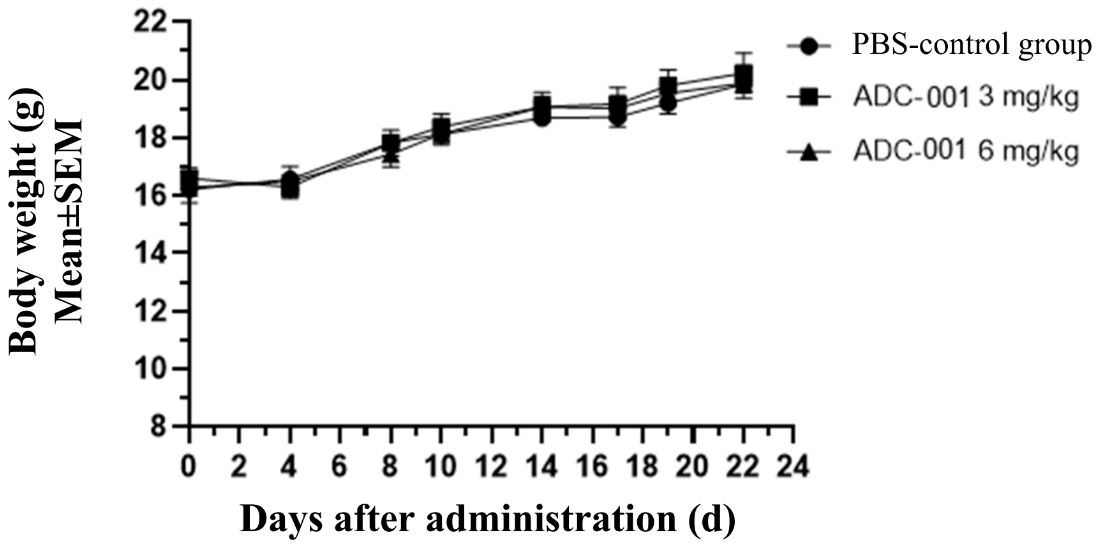


FIG. 2

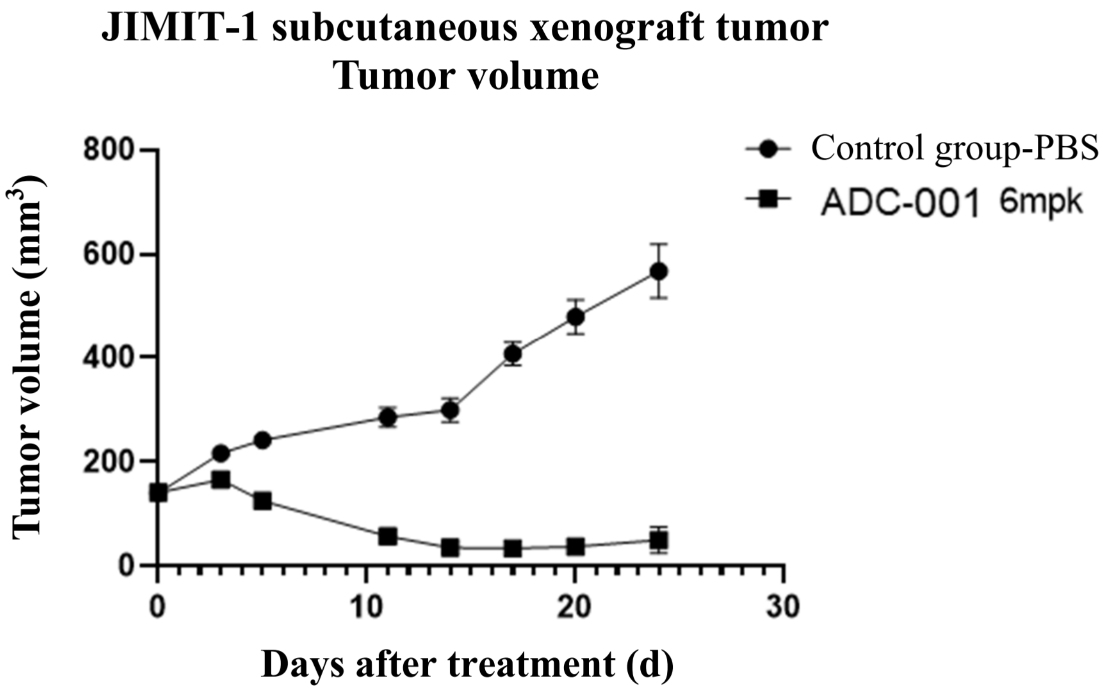
JIMIT-1 subcutaneous xenograft tumor
Tumor volume
Control group-PBS
ADC-001 6mpk
Tumor volume (mm3)
800
600
400
200
0
0
10
20
30
Days after treatment (d)

FIG. 3

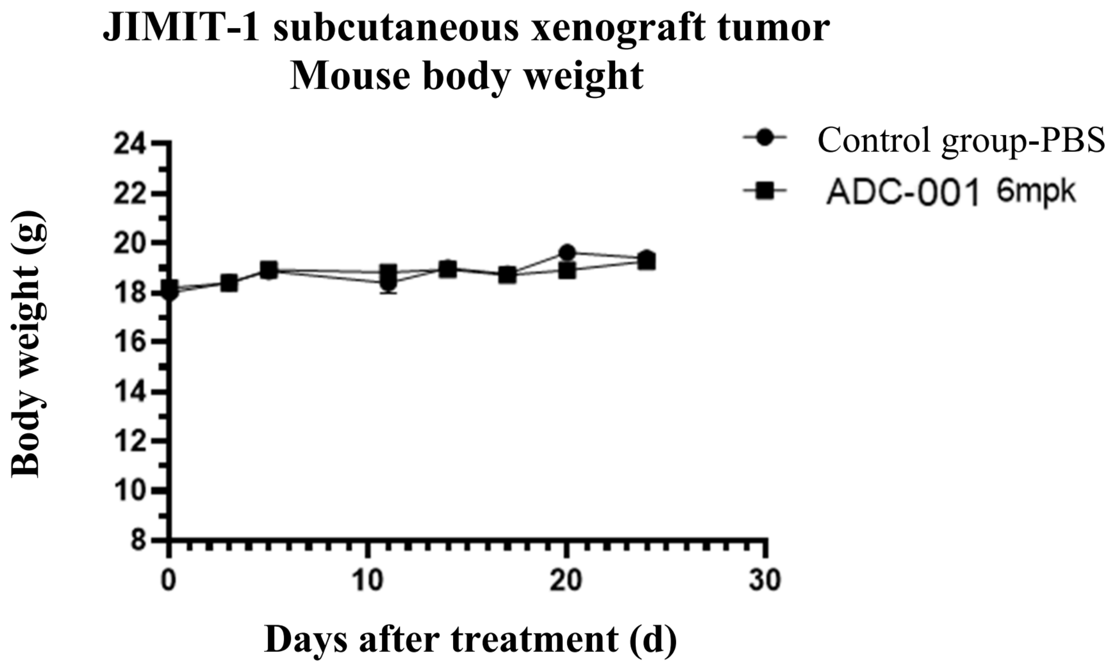
JIMIT-1 subcutaneous xenograft tumor
Mouse body weight
Control group-PBS
ADC-001 6mpk
Body weight (g)
24
22
20
18
16
14
12
10
8
0
10
20
30
Days after treatment (d)

FIG. 4

## INTERNATIONAL SEARCH REPORT

International application No.
**PCT/CN2024/073229**

**A. CLASSIFICATION OF SUBJECT MATTER**

A61K39/395(2006.01)i； A61K47/68(2017.01)i； A61K9/19(2006.01)i； A61K47/18(2017.01)i； A61P35/00(2006.01)i

According to International Patent Classification (IPC) or to both national classification and IPC

**B. FIELDS SEARCHED**

Minimum documentation searched (classification system followed by classification symbols)

IPC:A61K,A61P

Documentation searched other than minimum documentation to the extent that such documents are included in the fields searched

Electronic data base consulted during the international search (name of data base and, where practicable, search terms used)

CNTXT, WPABS, WPABSC, ENTXTC, OETXT, VEN, 中国药物专利数据库, China Medicine Patent Database, CNKI, 百度学术, BAIDU SCHOLAR, 超星读秀学术, DIUXIU SCHOLAR (CN), 万方, WANFANG (CN), Medline, ISI-WEB OF SCIENCE, REGISTRY, CAPLUS: 结构式检索, structural formula search, 帕妥珠单抗, 培妥珠单抗, 艾日布林, 缓冲, 抗体药物, 偶联物, 缀合物, antibody, drug, conjugate, pertuzumab, omnitarg, eribulin, buffer

**C. DOCUMENTS CONSIDERED TO BE RELEVANT**

| Category* | Citation of document, with indication, where appropriate, of the relevant passages | Relevant to claim No. |
|---|---|---|
| Y | WO 2021148003 A1 (SHANGHAI SENHUI MEDICINE CO., LTD. et al.) 29 July 2021 (2021-07-29)<br>see claims 1-46, and description, pages 18-25, 37-38, 42 and 79, embodiments 2-16 | 1-16 |
| Y | WO 2021190581 A1 (JIANGSU HENGRUI MEDICINE CO., LTD. et al.) 30 September 2021 (2021-09-30)<br>see abstract, claims 1-19, and description, pages 20-23 | 1-16 |
| Y | CN 113521018 A (NOVOCODEX BIOPHARMACEUTICALS CO., LTD.) 22 October 2021 (2021-10-22)<br>see abstract, and claims 1-10 | 1-16 |
| Y | CN 112516090 A (SHANGHAI FUDAN-ZHANGJIANG BIO-PHARMACEUTICAL CO., LTD. et al.) 19 March 2021 (2021-03-19)<br>see abstract, claims 1-10, and description, embodiments | 1-16 |

☑ Further documents are listed in the continuation of Box C.   ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **27 March 2024** | **12 April 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/073229**

**C. DOCUMENTS CONSIDERED TO BE RELEVANT**

| Category* | Citation of document, with indication, where appropriate, of the relevant passages | Relevant to claim No. |
|---|---|---|
| Y | CN 110732023 A (JIANGSU HENGRUI MEDICINE CO., LTD. et al.) 31 January 2020 (2020-01-31)<br>see abstract, and claims 1-18 | 1-16 |
| Y | CN 114828895 A (EISAI R. & D. MANAGEMENT CO., LTD.) 29 July 2022 (2022-07-29)<br>see abstract, and claims 1-8 | 1-16 |
| Y | CN 108883198 A (EISAI R. & D. MANAGEMENT CO., LTD.) 23 November 2018 (2018-11-23)<br>see abstract, and claims 1-169 | 1-16 |

Form PCT/ISA/210 (second sheet) (July 2022)

**INTERNATIONAL SEARCH REPORT**
**Information on patent family members**

International application No.
**PCT/CN2024/073229**

| Patent document cited in search report | Publication date (day/month/year) | Patent family member(s) | Publication date (day/month/year) |
|---|---|---|---|
| WO 2021148003 A1 | 29 July 2021 | AU 2021210079 A1 | 25 August 2022 |
| | | BR 112022014398 A2 | 13 September 2022 |
| | | MX 2022009044 A | 11 August 2022 |
| | | EP 4094779 A1 | 30 November 2022 |
| | | EP 4094779 A4 | 22 November 2023 |
| | | TW 202140078 A | 01 November 2021 |
| | | JP 2023511163 A | 16 March 2023 |
| | | KR 20220130160 A | 26 September 2022 |
| | | CA 3168654 A1 | 29 July 2021 |
| | | US 2023144203 A1 | 11 May 2023 |
| WO 2021190581 A1 | 30 September 2021 | MX 2022011770 A | 18 October 2022 |
| | | KR 20220157998 A | 29 November 2022 |
| | | BR 112022019073 A2 | 08 November 2022 |
| | | JP 2023518583 A | 02 May 2023 |
| | | AU 2021243073 A1 | 13 October 2022 |
| | | TW 202144013 A | 01 December 2021 |
| | | US 2023165969 A1 | 01 June 2023 |
| | | EP 4129345 A1 | 08 February 2023 |
| | | EP 4129345 A4 | 29 November 2023 |
| | | CA 3175733 A1 | 30 September 2021 |
| | | CN 115103691 A | 23 September 2022 |
| CN 113521018 A | 22 October 2021 | CN 113521018 B | 18 November 2022 |
| | | WO 2023000171 A1 | 26 January 2023 |
| CN 112516090 A | 19 March 2021 | CN 112516090 B | 20 June 2023 |
| CN 110732023 A | 31 January 2020 | CN 110732023 B | 16 June 2023 |
| CN 114828895 A | 29 July 2022 | WO 2021132166 A1 | 01 July 2021 |
| | | BR 112022012546 A2 | 06 September 2022 |
| | | IL 293040 A | 01 July 2022 |
| | | CA 3164797 A1 | 01 July 2021 |
| | | KR 20220120586 A | 30 August 2022 |
| | | MX 2022007798 A | 19 July 2022 |
| | | EP 4088739 A1 | 16 November 2022 |
| | | AU 2020414996 A1 | 14 July 2022 |
| | | JPWO 2021132166 A1 | 01 July 2021 |
| | | US 2023074385 A1 | 09 March 2023 |
| CN 108883198 A | 23 November 2018 | TW 202241524 A | 01 November 2022 |
| | | TWI 825834 B | 11 December 2023 |
| | | UA 125024 C2 | 29 December 2021 |
| | | JOP 20210074 A1 | 30 January 2023 |
| | | JP 2020019787 A | 06 February 2020 |
| | | JP 6870051 B2 | 12 May 2021 |
| | | CA 3013791 A1 | 08 September 2017 |
| | | US 2020297860 A1 | 24 September 2020 |
| | | EP 3824909 A1 | 26 May 2021 |
| | | US 2017252458 A1 | 07 September 2017 |
| | | US 10548986 B2 | 04 February 2020 |
| | | SG 10202007520 WA | 29 September 2020 |
| | | AU 2023285804 A1 | 18 January 2024 |
| | | AR 121302 A2 | 04 May 2022 |

Form PCT/ISA/210 (patent family annex) (July 2022)

**INTERNATIONAL SEARCH REPORT**
**Information on patent family members**

International application No.
**PCT/CN2024/073229**

| Patent document cited in search report | Publication date (day/month/year) | Patent family member(s) | Publication date (day/month/year) |
|---|---|---|---|
| | | LT 3423105 T | 10 September 2021 |
| | | IL 261428 A | 31 October 2018 |
| | | JP 2023082096 A | 13 June 2023 |
| | | SG 11201806515 RA | 27 September 2018 |
| | | CO 2018008667 A2 | 31 August 2018 |
| | | BR 112018067379 A2 | 15 January 2019 |
| | | RS 62108 B1 | 31 August 2021 |
| | | KR 20220101204 A | 19 July 2022 |
| | | MX 2023003809 A | 12 April 2023 |
| | | PE 20231049 A1 | 11 July 2023 |
| | | KR 20180115330 A | 22 October 2018 |
| | | KR 102445255 B1 | 22 September 2022 |
| | | HUE 054726 T2 | 28 September 2021 |
| | | CL 2021000048 A1 | 28 May 2021 |
| | | TW 201800110 A | 01 January 2018 |
| | | TWI 772288 B | 01 August 2022 |
| | | JP 2020128413 A | 27 August 2020 |
| | | JP 2019516664 A | 20 June 2019 |
| | | JP 6599019 B2 | 30 October 2019 |
| | | KR 20240007722 A | 16 January 2024 |
| | | MY 189113 A | 26 January 2022 |
| | | PL 3423105 T3 | 25 October 2021 |
| | | MD 3423105 T2 | 31 October 2021 |
| | | ES 2880402 T3 | 24 November 2021 |
| | | HRP 20211125 T1 | 15 October 2021 |
| | | JOP 20210073 A1 | 30 January 2023 |
| | | EP 3423105 A1 | 09 January 2019 |
| | | EP 3423105 B1 | 05 May 2021 |
| | | JOP 20170053 B1 | 17 August 2021 |
| | | IL 292946 A | 01 July 2022 |
| | | AR 107787 A1 | 06 June 2018 |
| | | WO 2017151979 A1 | 08 September 2017 |
| | | US 2018193478 A1 | 12 July 2018 |
| | | US 10322192 B2 | 18 June 2019 |
| | | AU 2017225982 A1 | 16 August 2018 |
| | | AU 2017225982 B2 | 05 October 2023 |
| | | PE 20181953 A1 | 17 December 2018 |
| | | SI 3423105 T1 | 31 December 2021 |
| | | PH 12018501847 A1 | 15 May 2019 |
| | | US 2023398228 A1 | 14 December 2023 |
| | | AR 121301 A2 | 04 May 2022 |
| | | CY 1124628 T1 | 22 July 2022 |
| | | DK 3423105 T3 | 26 July 2021 |
| | | MX 2018010562 A | 20 February 2019 |
| | | PE 20231050 A1 | 11 July 2023 |
| | | KR 20220101203 A | 19 July 2022 |
| | | KR 102456433 B1 | 19 October 2022 |
| | | CL 2018002456 A1 | 21 December 2018 |
| | | RU 2018134331 A | 02 April 2020 |
| | | RU 2018134331 A3 | 14 August 2020 |

Form PCT/ISA/210 (patent family annex) (July 2022)

**INTERNATIONAL SEARCH REPORT**
**Information on patent family members**

International application No.
**PCT/CN2024/073229**

| Patent document cited in search report | Publication date (day/month/year) | Patent family member(s) | | | Publication date (day/month/year) |
|---|---|---|---|---|---|
| | | RU | 2754369 | C2 | 01 September 2021 |
| | | PT | 3423105 | T | 19 July 2021 |
| | | MX | 2023003808 | A | 12 April 2023 |
| | | RU | 2021125492 | A | 05 April 2022 |
| | | MX | 2023003806 | A | 12 April 2023 |
| | | CL | 2021000049 | A1 | 28 May 2021 |
| | | MA | 45280 | A | 09 January 2019 |
| | | MA | 45280 | B1 | 31 August 2021 |
| | | JP | 2021185176 | A | 09 December 2021 |
| | | JP | 7254861 | B2 | 10 April 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050238649 A1
- US 5208020 A
- CN 2022107479 W
- WO 2017151979 A
- WO 2006033700 A


### Non-patent literature cited in the description


- **CHARI et al.** *Cancer Research*, 1992, vol. 52, 127-131
- Antibodies: A Laboratory Manual and Molecular Cloning: A Laboratory Manual. Cold Spring Harbor Laboratory